# EUROPEAN PATENT APPLICATION

(11) **EP 4 711 447 A1**
(43) Date of publication of application: **18.03.2026**
(21) Application number: 24803716.0
(22) Date of filing: 09.05.2024
(51) Int. Cl.: C12N 5/071, C12N 5/079, C12N 5/077

(54) **MEDIUM COMPOSITION FOR CULTURING NEUROMUSCULAR ORGANOID AND METHOD FOR PRODUCING NEUROMUSCULAR ORGANOID**

(30) Priority: 09.05.2023 KR 20230059675; 08.05.2024 KR 20240060292
(71) Applicant: Animuscure Inc., Suwon-si, Gyeonggi-do 16419 (KR)
(72) Inventor: LEE, Sang-Jin, Seoul 04747 (KR); LEE, Jinwoo, Suwon-si, Gyeonggi-do 16421 (KR)
(74) Representative: Rimini, Rebecca
(86) International application number: PCT/KR2024/006200
(87) International publication number: WO 2024/232663

(57) **Abstract**

The present disclosure relates to a medium composition for culturing a neuromuscular organoid, a manufacturing method for a neuromuscular organoid, etc. The neuromuscular organoid of the present disclosure is cultured in a medium containing a composition including a sonic hedgehog signaling agonist as an active ingredient, thereby promoting not only a process of differentiating neurons constituting the neuromuscular organoid into motor neurons, but also development and maturation of muscle fibers. The interactions between motor neurons and muscle tissues through a neuromuscular junction are essential for the cause and treatment of degenerative neuromuscular diseases, and thus, the neuromuscular organoid of the present disclosure can be used as a model of neuromuscular diseases (specifically, a degenerative neuromuscular disease model). Therefore, the present disclosure is expected to be applicable even to research on the mechanism of neurological and muscular diseases and to drug screening.

## Description

### Technical Field

The present disclosure relates to a medium composition for culturing a neuromuscular organoid and a manufacturing method for a neuromuscular organoid.

The present disclosure is based on and claims priority to Korean Patent Application No. 10-2023-0059675, filed on May 9, 2023, and Korean Patent Application No. 10-2024-0060292, filed on May 8, 2024, the disclosures of which are incorporated by reference herein in their entirety.

### Background Art

In the process of developing new drugs, animal testing on animals similar to humans is required prior to conducting clinical trials. Animals such as mice and rabbits are used in animal testing, but the ethics of this practice are a matter of concern. Furthermore, since animal testing models differ from humans in the genetic or biological characteristics, existing drug testing has limitations in the reliability of drug trial responses, such as finding of side effects in humans that were not found in animal testing.

To address these issues, various measures are being studied. However, conventional two-dimensional (2D) cell culture methods have the disadvantage of struggling to reproduce the inherent properties and characteristics of tissues. Xenograft models derived from cancer patients are sometimes used as an alternative, but have the disadvantages of being unsuitable for large-scale drug screening. Therefore, studies on experimental methods using organoids have been actively conducted in recent years.

Organoids are organ-like structures produced by culturing stem cells in a three-dimensional (3D) manner or recombining stem cells, and originate from various internal organs and contain stem cells therein, forming aggregates that can differentiate into cell bodies. Furthermore, organoids derived from each organ within the body have structures highly similar to the corresponding organs, and thus may be used as models to replace cellular and animal experiments.

Meanwhile, research on organoids is entering a stage where it is about to establish systems applicable to clinical research, moving beyond basic research for the development. However, compared to organoids of the brain and intestines, which have relatively simple structures, research on neuromuscular organoids composed of two distinct tissues are very limited (the number of relevant papers published over the past 10 years registered in the PubMed database: brain organoids (1749 papers), intestinal organoids (2295 papers), and neuromuscular organoids (66 papers)). In addition, at the Max Delbruck Center for Molecular Medicine in Germany, human induced pluripotent stem cells were induced to differentiate into bipotent neuromesodermal progenitors (NMPs), which can differentiate both neurons and skeletal muscle cells to form aggregates, and by self-organization, neuromuscular organoids composed of nervous tissue and skeletal muscle tissue were developed (see Self-organizing 3D Human Trunk Neuromuscular Organoids, Cell Stem Cell, 2020). However, only about 6 % of neurons constituting an organoid were confirmed to differentiate into motor neurons.

Moreover, motor neurons cause contraction of muscle tissue through a neuromuscular junction, and apoptosis of motor neurons is a representative symptom of degenerative neuromuscular diseases. Interactions between motor neurons and muscle tissue through the neuromuscular junction are fundamental to the cause and treatment of degenerative neuromuscular diseases. To be used as a model for a degenerative neuromuscular disease, the model should contain all of motor neurons, skeletal muscle tissue, and the neuromuscular junction connecting these two tissues.

Therefore, there is a need to optimize neuromuscular organoids composed of motor neurons, which can be utilized for studying mechanisms of neuromuscular diseases (specifically, degenerative neuromuscular diseases) and for drug screening.

### Disclosure of Invention

### Technical Goal of the Invention

An object of the present disclosure is to provide a medium composition for culturing a neuromuscular organoid, for three-dimensional culture of pluripotent stem cells (PSCs) into a neuromuscular organoid, the medium composition including a sonic hedgehog (SHH) signaling activator as an active ingredient.

Another object of the present disclosure is to provide a manufacturing method for a neuromuscular organoid, the method including the following processes:
(S0) culturing subject-derived human pluripotent cells (hPSCs) in a medium containing glycogen synthase kinase 3 (GSK-3) inhibitor and fibroblast growth factor (FGF) to differentiate into bipotent neuromesodermal progenitors (NMPs);
(S1) diluting the bipotent NMPs, followed by dispensing, to form embryoid bodies; and
(S2) culturing the embryoid bodies in a medium containing a sonic hedgehog (SHH) signaling activator to manufacture a three-dimensional neuromuscular organoid.

Another object of the present disclosure is to provide a three-dimensional neuromuscular organoid manufactured by the manufacturing method.

Another object of the present disclosure is to provide a neuromuscular disease model including the neuromuscular organoid.

Another object of the present disclosure is to provide a screening method for a therapeutic agent for a neuromuscular disease, the method including the following processes:
(1) treating a neuromuscular organoid derived from a neuromuscular disease subject manufactured by the manufacturing method; and
(2) comparing morphological characteristics of the neuromuscular organoid treated with the candidate substance.

However, technical problems to be solved by the present disclosure are not limited to the aforementioned problems, and other problems not mentioned will be clearly understood by those skilled in the art from the descriptions below.

### Solution to Problem

The present disclosure provides a medium composition for culturing a neuromuscular organoid, for three-dimensional culture of pluripotent stem cells (PSCs) into a neuromuscular organoid, the medium composition including a sonic hedgehog (SHH) signaling activator as an active ingredient.

In an embodiment of the present disclosure, the SHH signaling activator may be at least one selected from the group consisting of an SHH signaling agonist and an SHH protein, but the embodiment is not limited thereto.

In another embodiment of the present disclosure, the SHH signaling agonist may be at least one selected from the group consisting of purmorphamine and a smoothened agonist (SAG), but the embodiment is not limited thereto.

In another embodiment of the present disclosure, the medium composition may further contain at least one selected from the group consisting of glycogen synthase kinase 3 (GSK-3) inhibitor, fibroblast growth factor (FGF), Y-27632, insulin-like growth factor (IGF), and hepatocyte growth factor (HGF), but the embodiment is not limited thereto.

In another embodiment of the present disclosure, the neuromuscular organoid may include at least one selected from the group consisting of nervous tissue, skeletal muscle tissue, and a neuromuscular junction, but the embodiment is not limited thereto.

In another embodiment of the present disclosure, the pluripotent stem cell (PSC) may be a human induced pluripotent stem cell (hiPSC), but the embodiment is not limited thereto.

The present disclosure provides a manufacturing method for a neuromuscular organoid, the method including the following processes:
(S0) culturing hPSCs derived from a subject in a medium containing GSK-3 inhibitor and FGF to differentiate into bipotent neuromesodermal progenitors (NMPs);
(S1) diluting the bipotent NMPs, followed by dispensing, to form embryoid bodies; and
(S2) culturing the embryoid body in a medium containing an SHH signaling activator to produce a three-dimensional neuromuscular organoid.

In an embodiment of the present disclosure, in process (S0), the hPSCs may be in a colony state, but the embodiment is not limited thereto.

In another embodiment of the present disclosure, in process (S0), the bipotent NMPs may express SRY sex-determining region Y-box 2 (SOX2) or BRACHYURY (BRA), but the embodiment is not limited thereto.

In another embodiment of the present disclosure, in process (S1), a medium composition for diluting the bipotent NMPs may contain at least one selected from the group consisting of Y-27632, FGF, IGF, and HGF, but the embodiment is not limited thereto.

In another embodiment of the present disclosure, process (S1) may be, although not limited thereto, characterized by at least one selected from the following group consisting of:
(a) performing process (S1) before the bipotent NMPs form the embryoid body; and
(b) dispensing 1500 to 9000 bipotent NMPs.

In another embodiment of the present disclosure, in process (S2), the culturing may be orbital shaking culturing, but the embodiment is not limited thereto.

The present disclosure provides a three-dimensional neuromuscular organoid produced by the method.

In an embodiment of the present disclosure, the organoid may express at least one selected from the group consisting of neuron-specific class III beta-tubulin (TUJ1), fast myosin heavy chain (MHC), SOX2, SHH, α-bungarotoxin (α-BTX), myosin heavy chain 2 (MYH2), and myosin heavy chain 7 (MYH7), but the embodiment is not limited thereto.

In another embodiment of the present disclosure, the neuromuscular organoid may be, although not limited thereto, characterized by at least one selected from the following group consisting of:
(a) decreased expression of Cdo and Tubb3; and
(b) increased expression of olig2 and choline acetyltransferase (ChAT).

In another embodiment of the present disclosure, the neuromuscular organoid may be, although not limited thereto, characterized by at least one selected from the following group consisting of:
(a) promoting differentiation into motor neurons;
(b) increasing the size of muscle fibers; and
(c) maturing muscle fibers.

In another embodiment of the present disclosure, the neuromuscular organoid may exhibit synchronous contraction, but the embodiment is not limited thereto.

The present disclosure provides a neuromuscular disease model including the neuromuscular organoid.

In an embodiment of the present disclosure, the neuromuscular disease may be a degenerative neuromuscular disease, but the embodiment is not limited thereto.

In another embodiment of the present disclosure, the degenerative neuromuscular disease may be selected from the group consisting of spinal muscular atrophy (SMA), amyotrophic lateral sclerosis (ALS), Duchenne muscular dystrophy, and myotonic dystrophy, but the embodiment is not limited thereto.

The present disclosure provides a screening method for a therapeutic agent for a neuromuscular disease, the method including the following processes:
(1) treating a neuromuscular disease candidate substance with a neuromuscular organoid derived from a neuromuscular disease subject produced by the method; and
(2) comparing morphological characteristics of the neuromuscular organoid treated with the candidate substance.

In an embodiment of the present disclosure, the screening method may further include (3) determining the candidate substance as a therapeutic agent for a neuromuscular disease, when the morphological characteristics of the neuromuscular organoid treated with the candidate substance exhibit morphological characteristics of normal neuromuscular tissue, but the embodiment is not limited thereto.

In addition, the present disclosure provides use of a medium composition for three-dimensional culture of a neuromuscular organoid from PSCs, the medium composition including an SHH signaling activator as an active ingredient.

In addition, the present disclosure provides use of a composition for manufacturing a neuromuscular organoid from PSCs, the composition including an SHH signaling activator as an active ingredient.

In addition, the present disclosure provides use of a three-dimensional neuromuscular organoid for screening for a therapeutic agent for a neuromuscular disease.

### Advantageous Effects of Invention

A neuromuscular organoid of the present disclosure is cultured in a medium containing a composition including a sonic hedgehog signaling agonist as an active ingredient, thereby not only promoting a process of differentiating neurons constituting the neuromuscular organoid into motor neurons, but also promoting the development and maturation of muscle fibers. The interaction between motor neurons and muscle tissues through a neuromuscular junction is essential for the cause and treatment of degenerative neuromuscular diseases, and thus, the neuromuscular organoid of the present disclosure can be used as a model of neuromuscular diseases (specifically, a degenerative neuromuscular disease model). Therefore, the present disclosure is expected to be applicable to mechanism studies and drug screening for neurological and muscular diseases.

### Brief Description of the Drawings

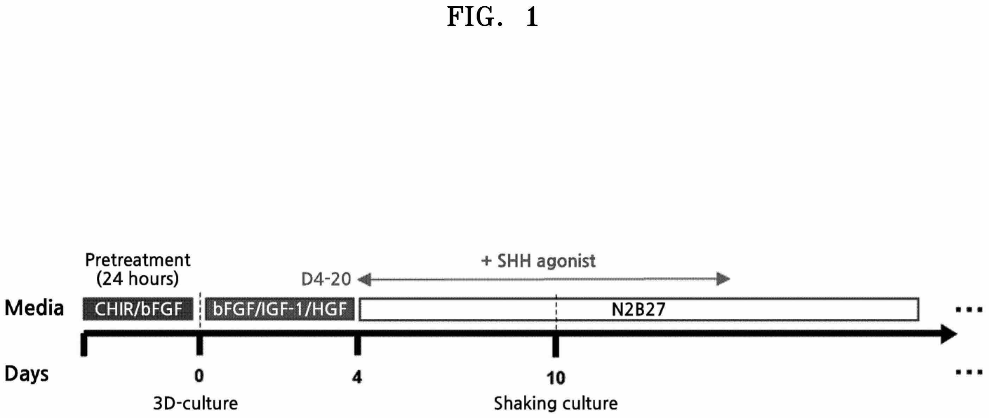

FIG. 1 is a diagram illustrating a protocol for producing a neuromuscular organoid.

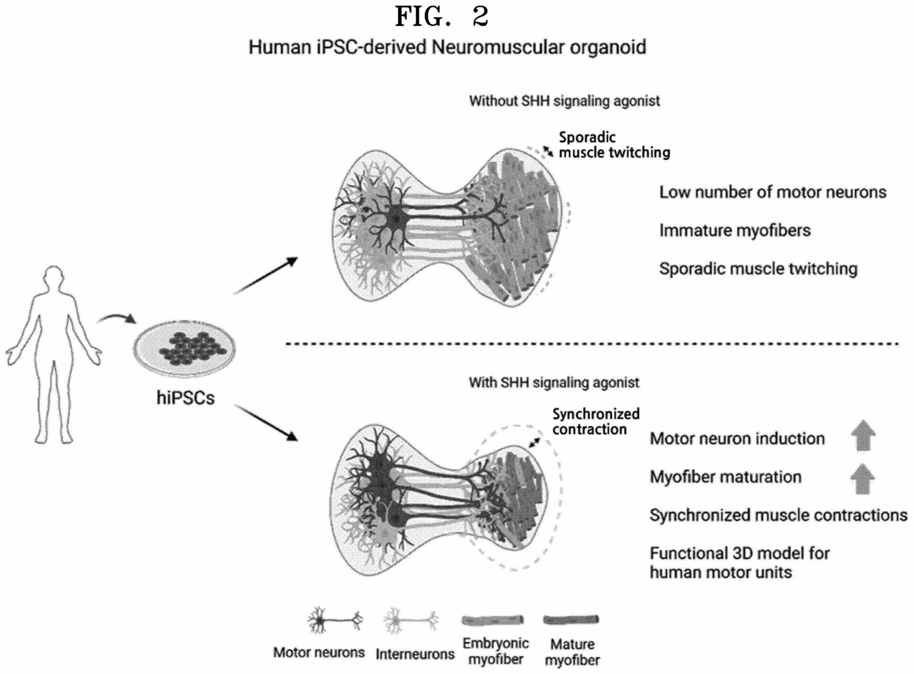

FIG. 2 is a diagram schematically illustrating changes in a neuromuscular organoid following treatment with a sonic hedgehog signaling agonist.

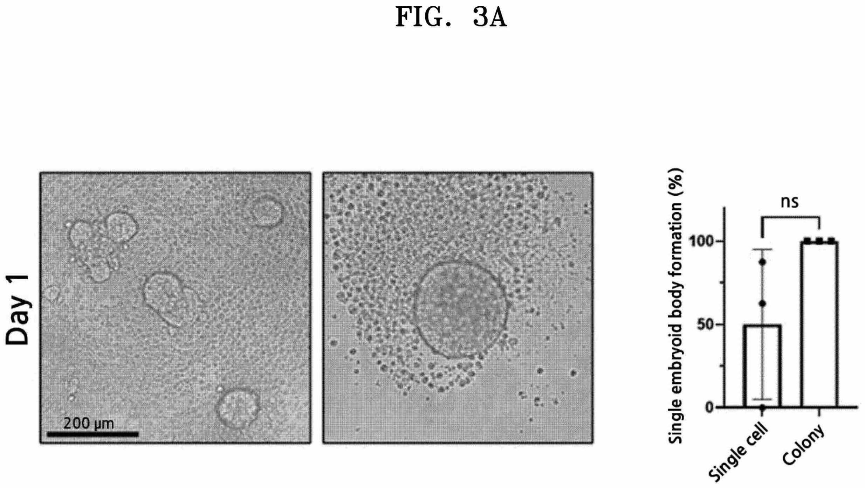

FIG. 3A shows representative images of embryoid body formation induced according to a pretreatment method, showing failed aggregation (left), successful embryoid body formation (middle), and successful embryoid body formation ratios according to a pretreatment method (right).

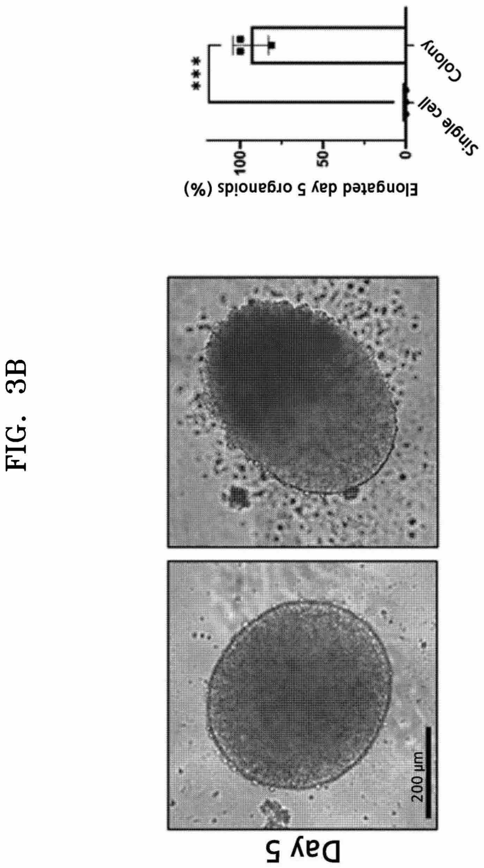

FIG. 3B shows representative images of neuromuscular morphogenesis induction based on elongation, showing failed morphogenesis (left), successful neuromuscular morphogenesis based on elongation (middle), and successful morphogenesis ratios according to a pretreatment method (right).

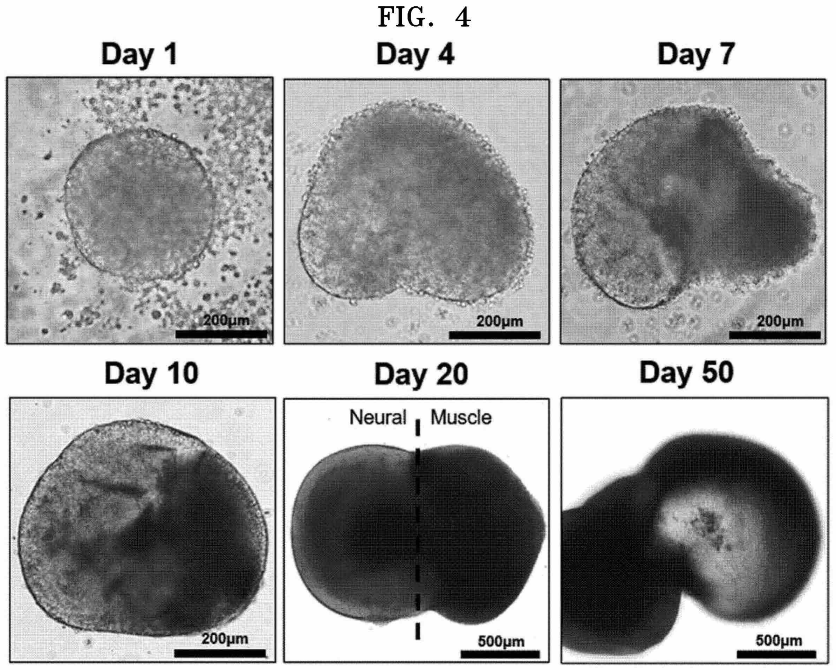

FIG. 4 is a diagram confirming successful formation of a neuromuscular organoid through observation of morphogenesis over time.

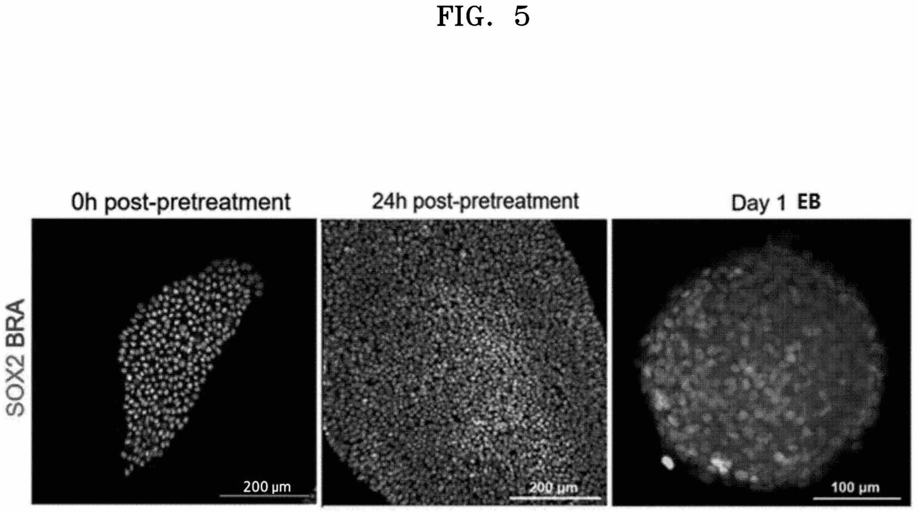

FIG. 5 shows that human pluripotent stem cells differentiated into bipotent neuromesodermal progenitors (NMPs) through a pretreatment method, as confirmed by the expression of SOX2 and BRACHYURY (BRA), which are NMP markers, wherein the induction into NMPs through a pretreatment method was confirmed by the simultaneous expression of SOX2 and BRA by immunofluorescence starting 24 hours after the pretreatment.

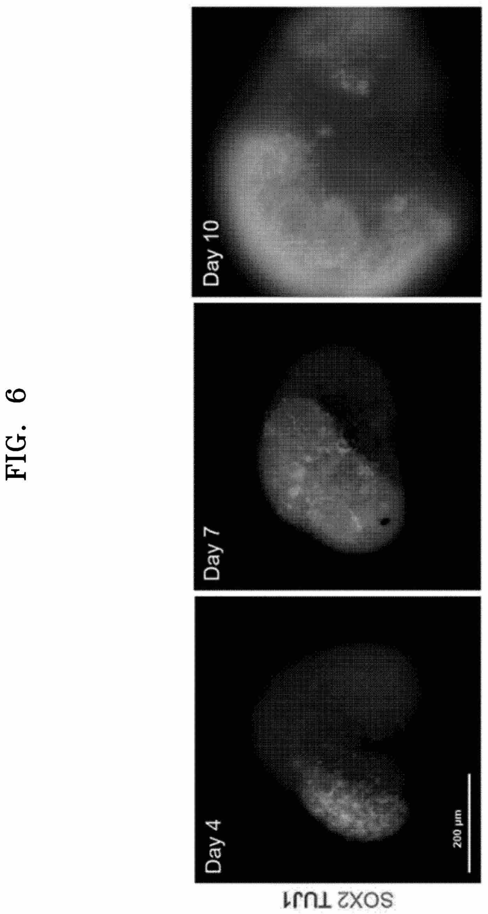

FIG. 6 shows immunofluorescence results for a neural stem cell marker (e.g., SOX2) and a neuron (e.g., neuron-specific class III beta-tubulin (TUJ1)) marker present in a neuromuscular organoid during various time periods, wherein, on Day 4 of the organoid formation, SOX2 was confirmed to be locally expressed in a site where nervous tissue is formed, indicating the formation of neuroectoderm (left), and starting Day 7 of the organoid formation, the differentiation of neural stem cells into neurons was confirmed by the expression of TUJ1 (middle).

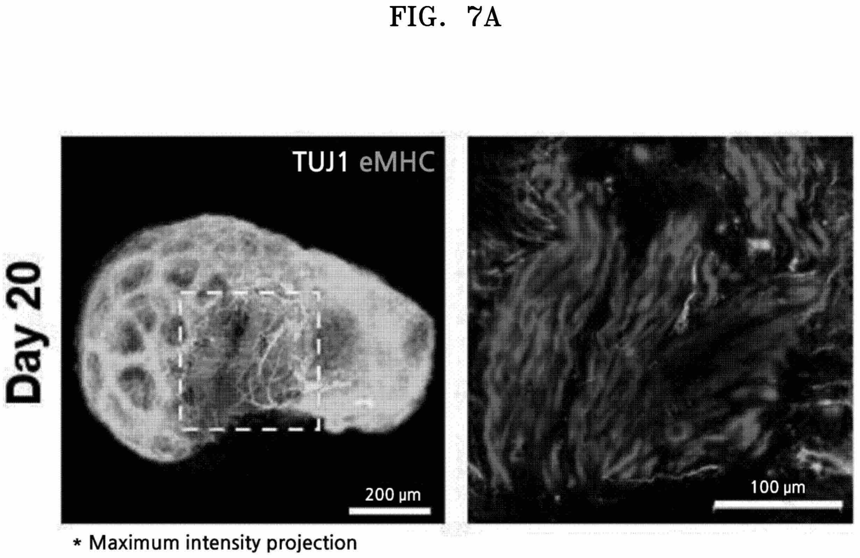

FIG. 7A shows immunofluorescence results for a neuronal marker and a skeletal muscle marker in a successful neuromuscular organoid (on Day 20), wherein the left image shows a neuromuscular organoid expressing a neuronal marker (i.e., TUJ1) and an embryonic myosin heavy chain isoform (eMHC), and the right panel shows a higher magnification image of the area in the left panel indicated by the dashed box.

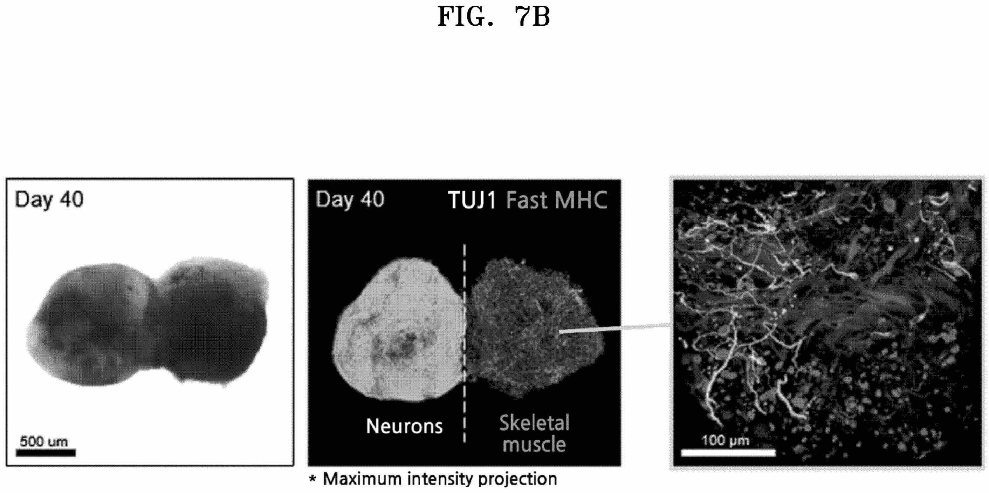

FIG. 7B shows immunofluorescence results for a neuronal marker and a skeletal muscle marker in a successful neuromuscular organoid (on Day 40), indicating that the neuromuscular organoid was successfully formed using the manufacturing method of the present disclosure. Specifically, the neuromuscular organoid expressing a neuronal cell marker (e.g., TUJ1) and a matured skeletal muscle (e.g., fast myosin heavy chain (MHC)) marker is depicted in the central diagram. In addition, the right panel shows a higher magnification image of the skeletal muscle site of the neuromuscular organoid, indicating that axons extend from the nervous tissue into the skeletal muscle tissue to form a neuromuscular junction.

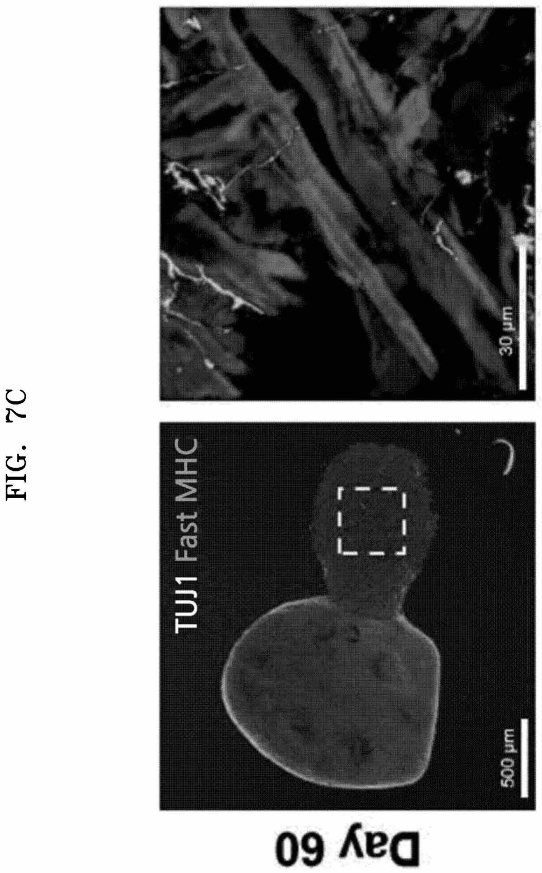

FIG. 7C shows immunofluorescence results for a neuronal marker and a skeletal muscle marker in a successful neuromuscular organoid (on Day 60), wherein the left image shows a neuromuscular organoid expressing a neuronal marker (e.g., TUJ1) and a matured skeletal muscle (e.g., fast MHC) marker, and the right panel shows a higher magnification image of the area in the left panel indicated by the dashed box.

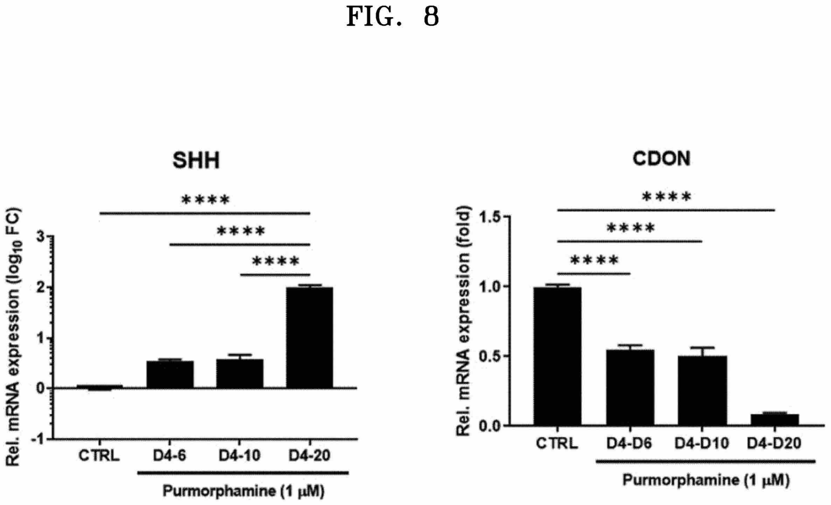

FIG. 8 is a diagram showing that sonic hedgehog (SHH) signaling was activated in a neuromuscular organoid by treatment with purmorphamine, as demonstrated by qRT-PCR results. (The expression values were normalized to the L32 level, and further normalized to the values of a control organoid. Error bars represent standard deviation. Data were analyzed using one-way ANOVA), followed by Tukey multiple comparison test (**** P < 0.0001).)

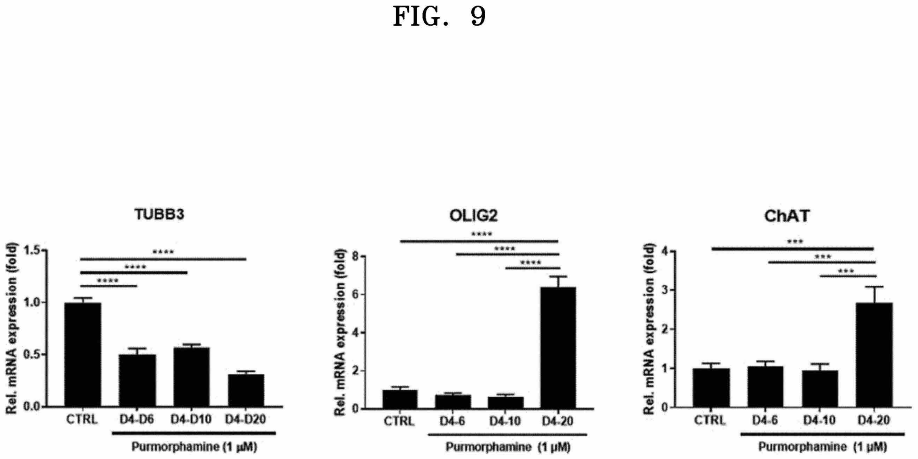

FIG. 9 shows results confirmed by the expression levels of motor neuron markers, indicating that differentiation into motor neurons was induced by treatment with purmorphamine, based on qRT-PCR analysis results for a motor neuron precursor cell marker (e.g., OLIG2), a motor neuron marker (e.g., ChAT), and a general neuronal marker (e.g., TUBB2). (The expression values were normalized to the L32 level, and further normalized to the values of a control organoid. Error bars represent standard deviation. Data were analyzed using one-way ANOVA), followed by Tukey multiple comparison test (*** P < 0.001, **** P < 0.0001).)

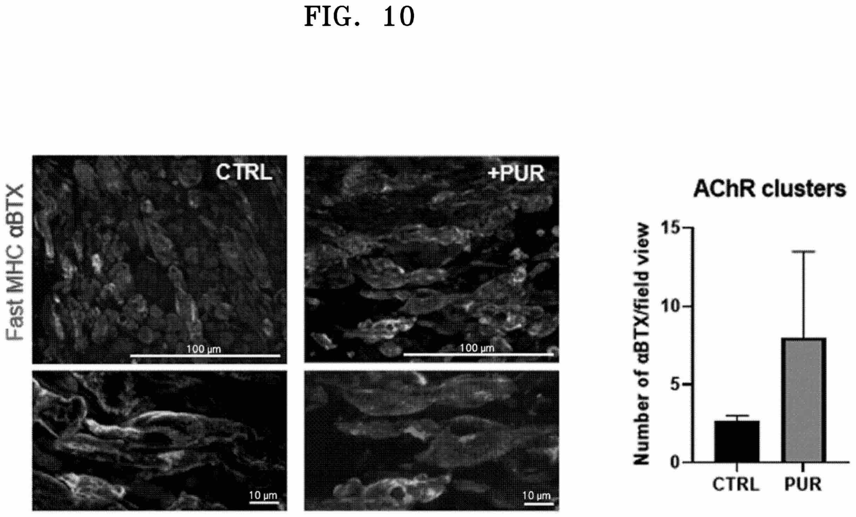

FIG. 10 is a diagram showing the frequency of neuromuscular junction formation following treatment with purmorphamine, as determined using α-bungarotoxin (α-BTX).

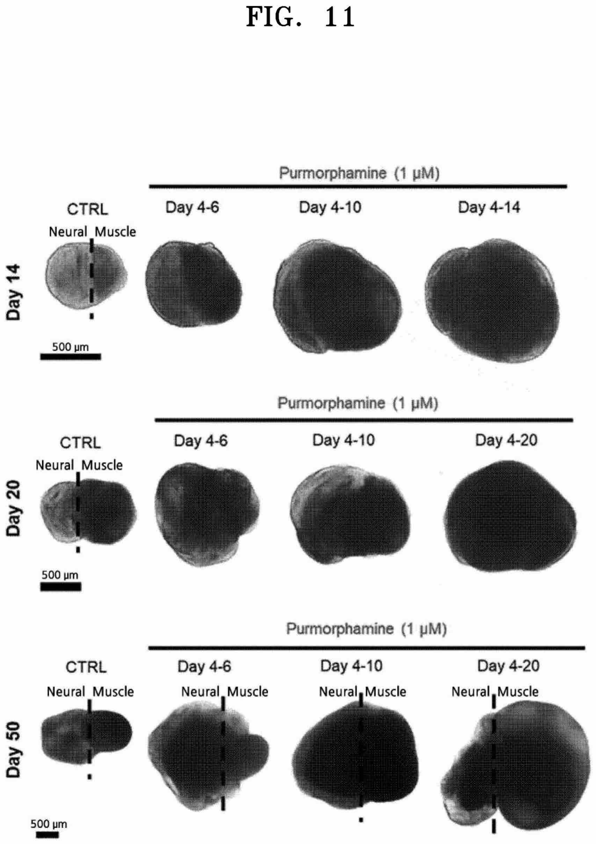

FIG. 11 shows the morphological differences in organoids according to the duration of treatment, following addition of purmorphamine to a culture medium from Day 4 of neuromuscular organoid formation, indicating that the size of the organoids generally increases with prolonged treatment with purmorphamine, particularly in the muscle tissue region.

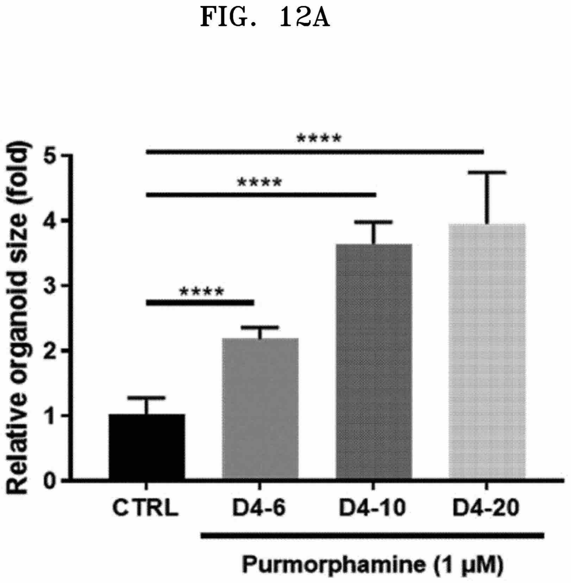

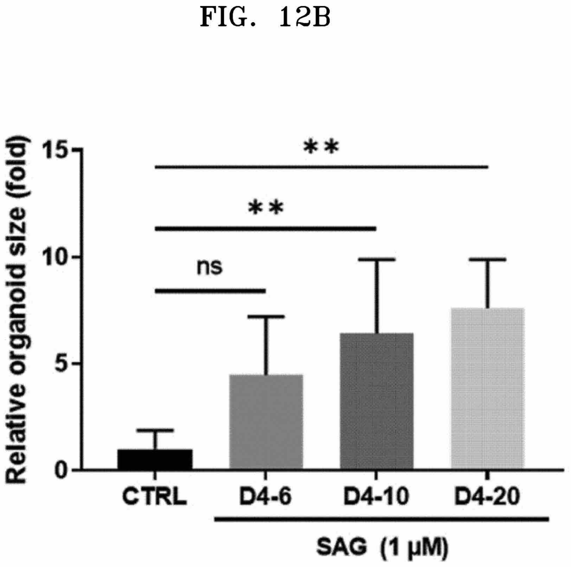

FIGS. 12A and 12B are diagrams for quantifying the size of neuromuscular organoids following treatment with an SHH signaling agonist (e.g., purmorphamine and SAG), demonstrating that, compared to a control group, the size of neuromuscular organoids increases when treated with the SHH signaling agonist. (The size of neuromuscular organoids on Day 20 after treatment with purmorphamine was measured by the area of the brightfield image, error bars represent standard deviation, and data were analyzed using one-way ANOVA), followed by Tukey multiple comparison test (**** P < 0.0001).)

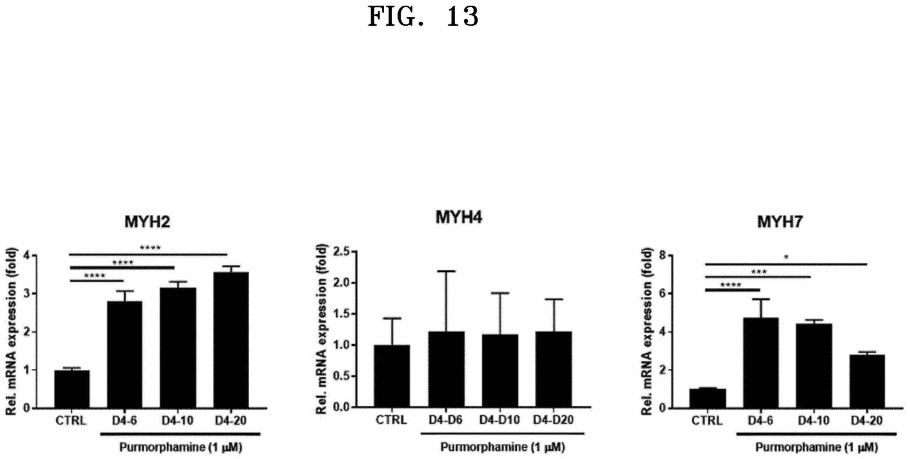

FIG. 13 is a diagram showing that treatment with purmorphamine increases the expression of mature muscle fiber markers, as demonstrated by qRT-PCR results. (The expression values were normalized to the L32 level, and further normalized to the values of a control organoid. Error bars represent standard deviation. Data were analyzed using one-way ANOVA), followed by Tukey multiple comparison test (* P < 0.05, *** P < 0.001, **** P < 0.0001).)

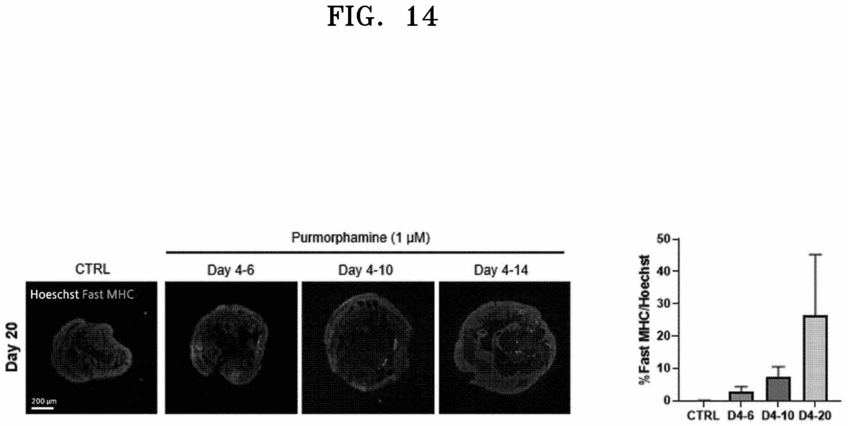

FIG. 14 is a diagram showing fast MHC, which is expressed specifically in mature muscle fibers, is expressed earlier compared to a control group.

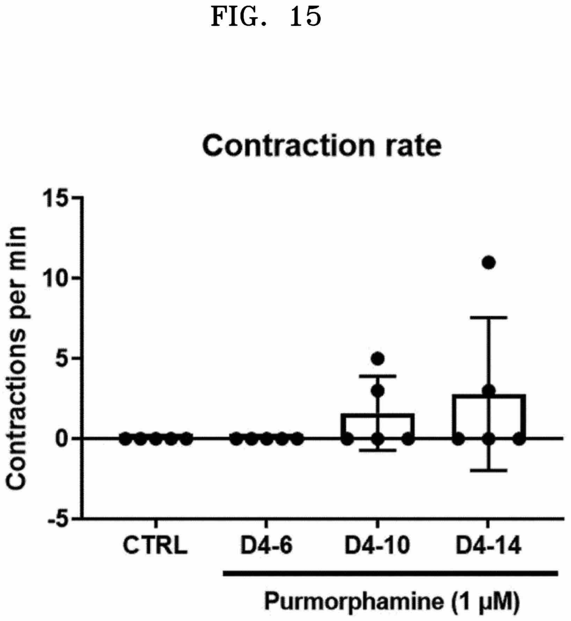

FIG. 15 is a diagram showing that synchronous contractions of muscle tissue induced by treatment with glutamate are observed early, compared to a control group, on Day 14 of neuromuscular organoid formation, as the maturation of muscle tissue is promoted.

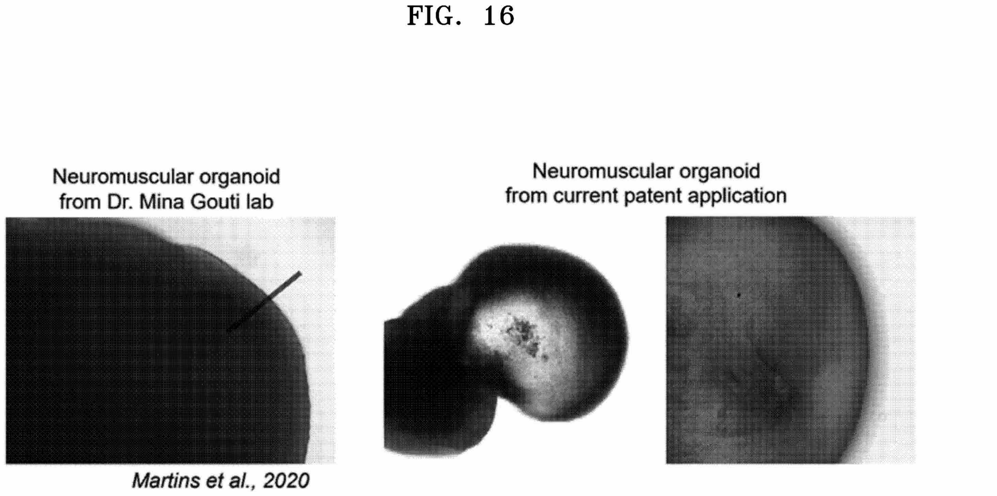

FIG. 16 is a diagram showing that, compared to the spontaneous contractions observed in neuromuscular organoids according to the prior art, neuromuscular organoids according to the present disclosure exhibit synchronized contractions of the entire skeletal muscle tissue that remarkably resembles the contractions of actual human muscles.

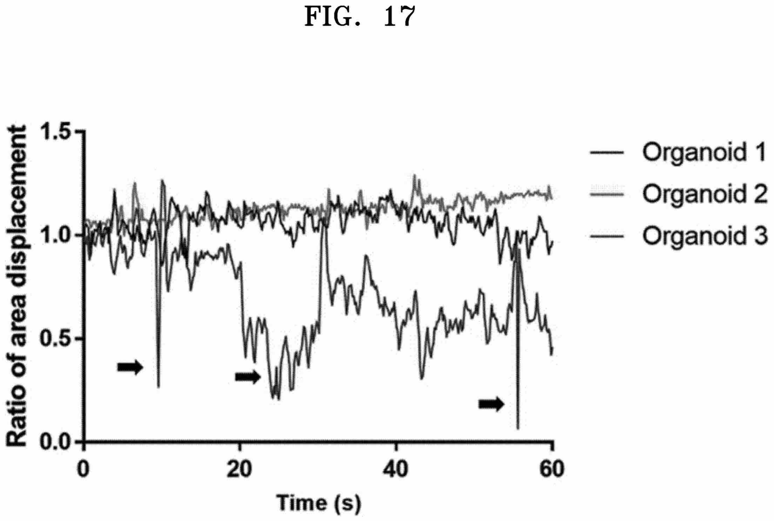

FIG. 17 is a diagram showing the quantified results of defining a region of interest in a video of a neuromuscular organoid on Day 50 and measuring area displacement over time, showing that synchronized contractions of the entire skeletal muscle tissue, which were not observed in neuromuscular organoids according to the prior art, were observed in neuromuscular organoids according to the present disclosure. Specifically, results of comparing contraction patterns of three different neuromuscular organoids are shown in the diagram.

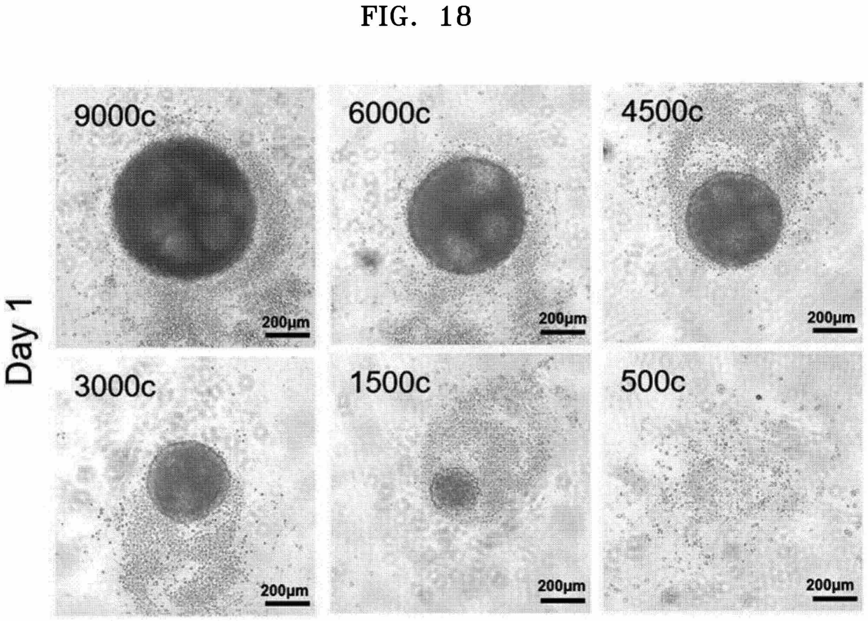

FIG. 18 is a diagram showing results of comparing the size of an embryoid body according to the number of starting cells. When the number of starting cells exceeds 4,500 (top three panels), the embryoid body was so large that voids were formed inside, when the number of starting cells is too low (500 cells, bottom right panel), NMPs failed to form an embryoid body, and the number of starting cells in a range of 3000 (bottom left panel) to 1500 (bottom middle panel) results in the formation of a well-condensed embryoid body.

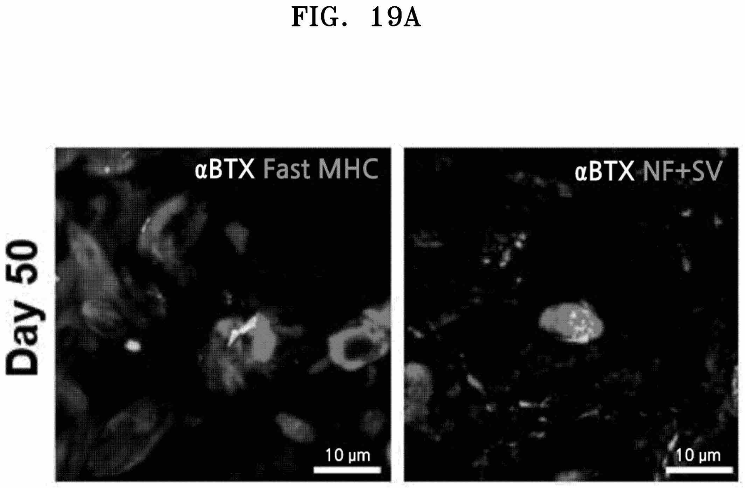

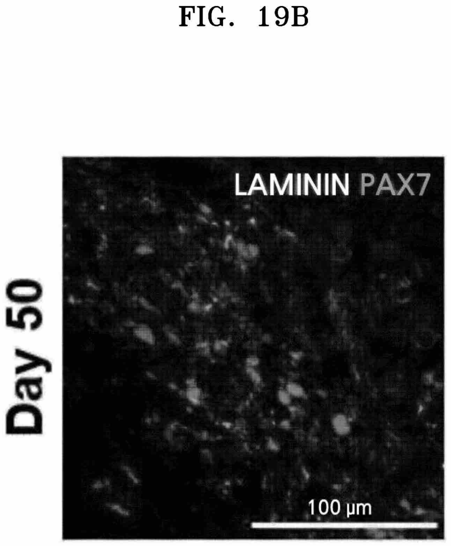

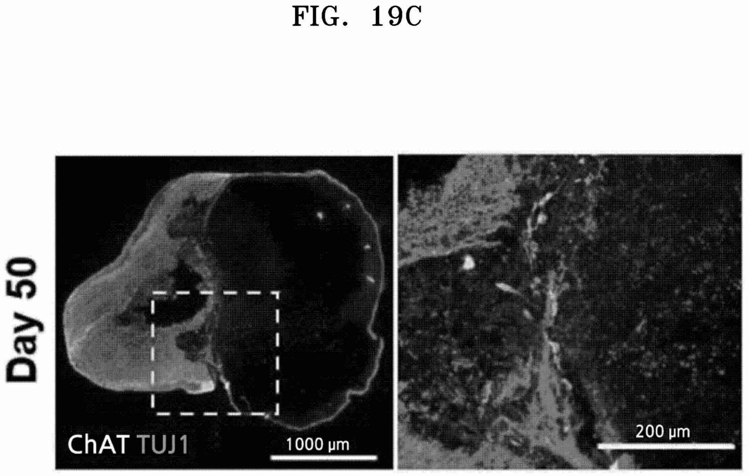

FIGS. 19A to 19C show immunofluorescence results demonstrating cell type diversity of neuromuscular organoids. FIG. 19A shows acetylcholine receptor clusters labeled with α-BTX using fast MHC (left) and a combination of neurofilament (NF) and synaptophysin (SV) (NF+SV, right). FIG. 19B shows a satellite cell marker (e.g., PAX7) labeled with LAMININ. FIG. 19C shows a motor neuron marker (e.g., ChAT) indicating a subset of neurons (e.g., TUJ1), wherein the right panel shows a higher magnification image of the area in the left panel indicated by the dashed box.

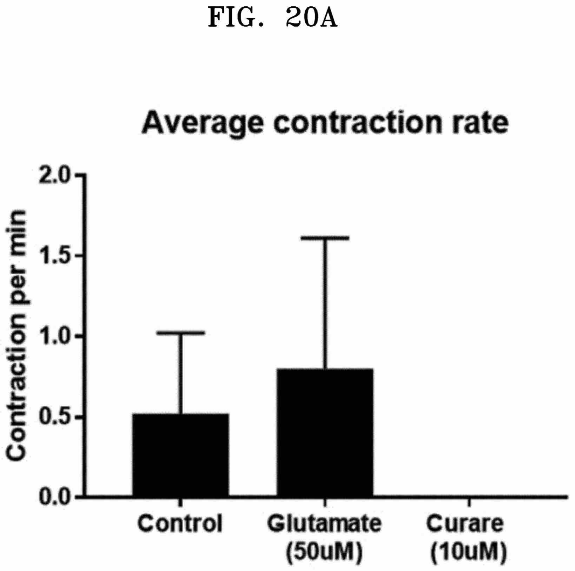

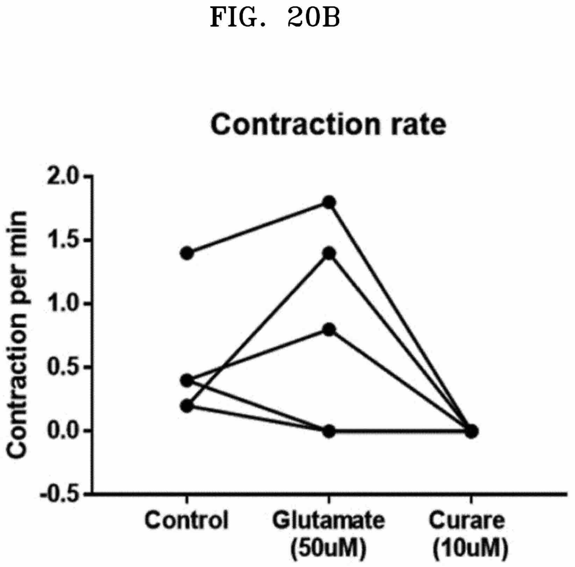

FIGS. 20A and 20B show results of synchronous contractions of skeletal muscle formed in neuromuscular organoids. The synchronous contractions of skeletal muscle formed in neuromuscular organoids on Day 50 were tracked via live imaging, with contraction rates displayed as averages (FIG. 20A) or individually (FIG. 20B), wherein changes in synchronous contraction rates were also recorded after sequentially treating organoids with glutamate and curare, with error bars indicating standard deviation (n=5).

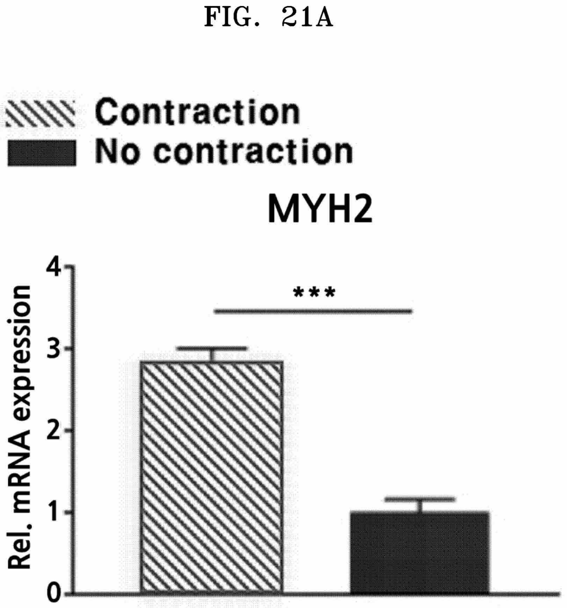

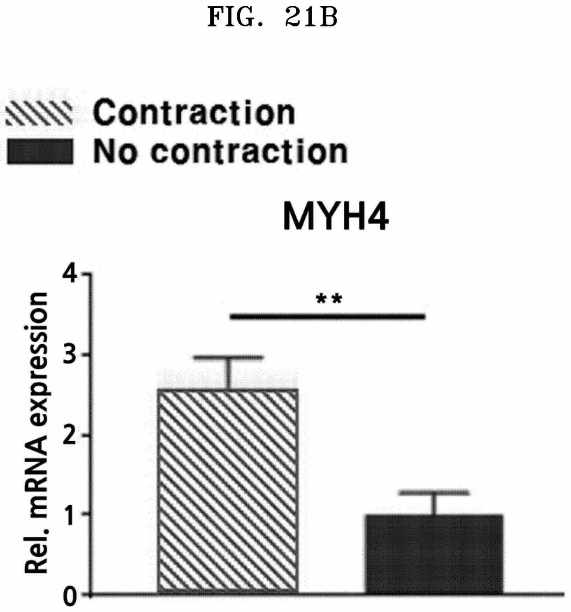

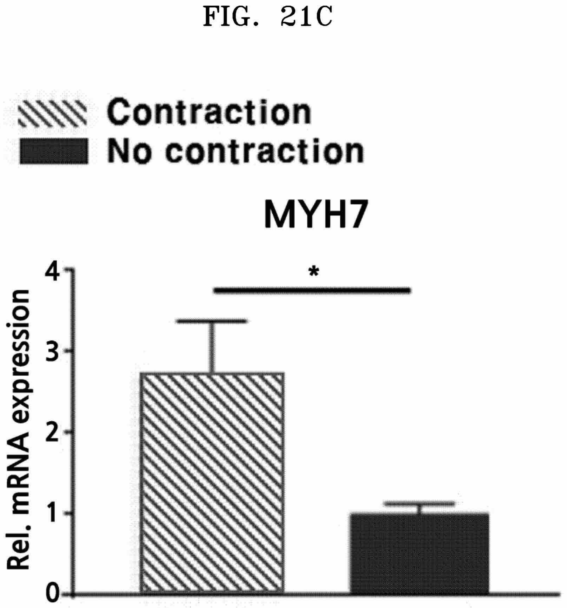

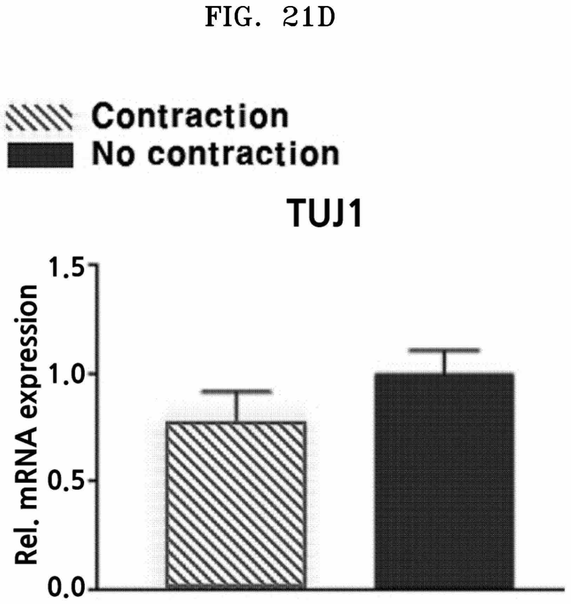

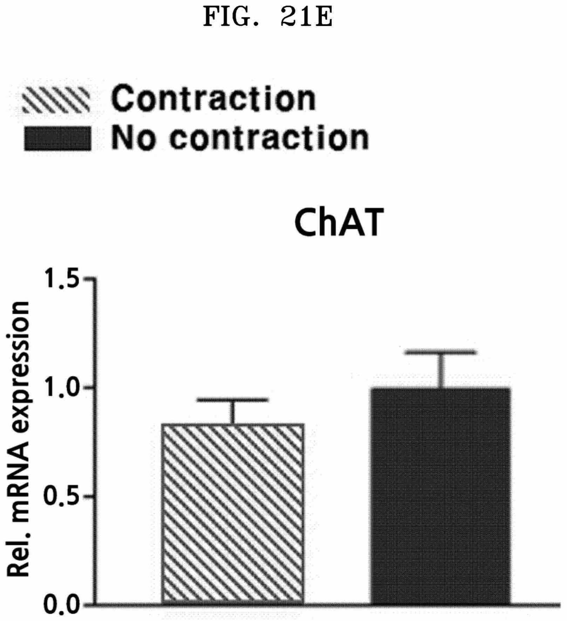

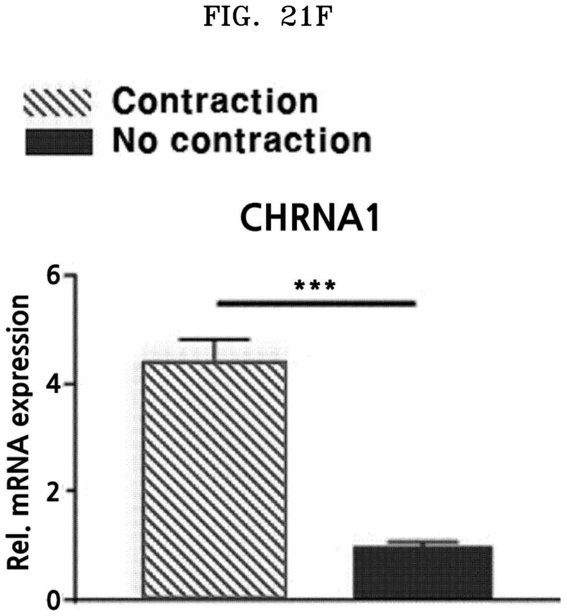

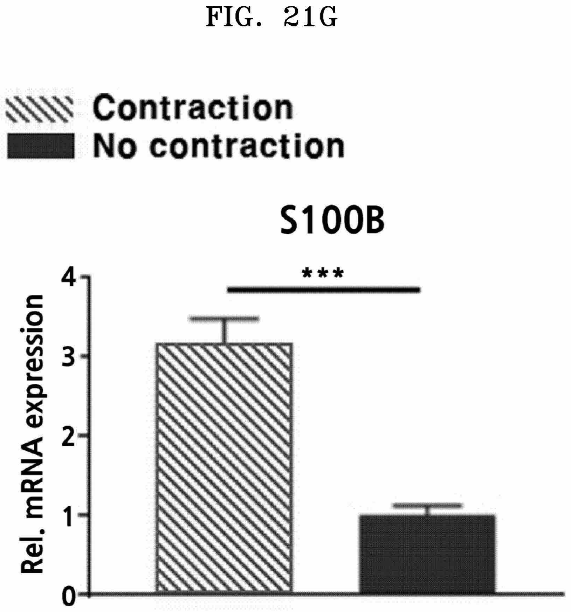

FIGS. 21A to 21G show results comparing neuromuscular organoids that are synchronously contractile and non-contractile. The qRT-PCR analysis results for myosin heavy chain isoforms (FIGS. 21A to 21C), neural markers (FIGS. 21D and 21E), and neuromuscular junction-related genes (FIGS. 21F and 21G) are shown. (The expression values were normalized to the L32 level, and further normalized to the values of non-contractile organoids. Error bars represent standard deviation, and statistical significance was calculated using Student t-test (paired, two-tailed) (* P < 0.05, ** P < 0.01, *** P < 0.001).)

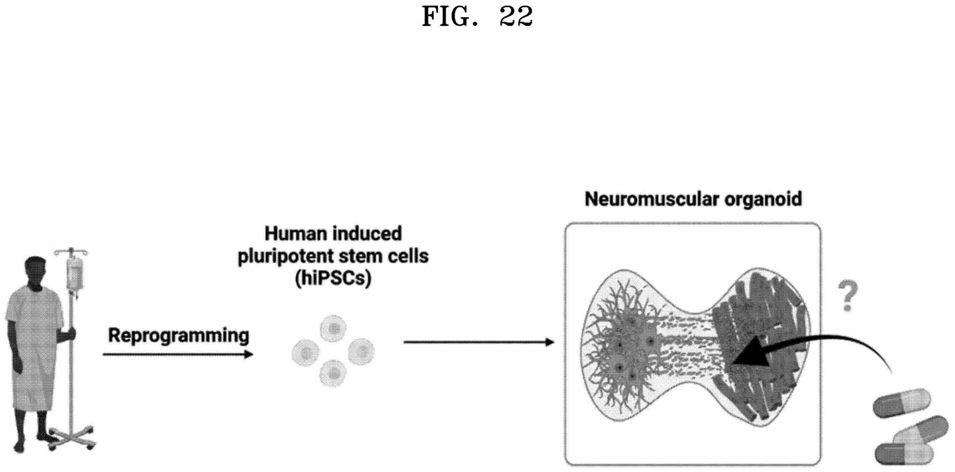

FIG. 22 is a schematic diagram illustrating utilization of a neuromuscular organoid in personalized medicine.

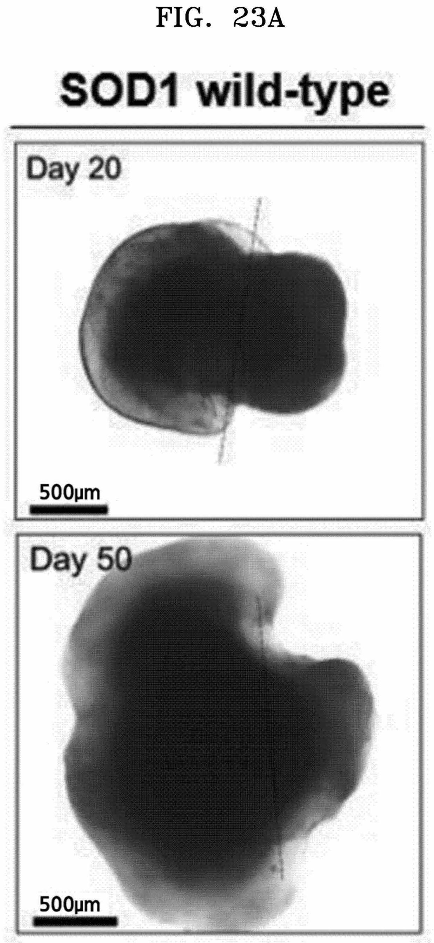

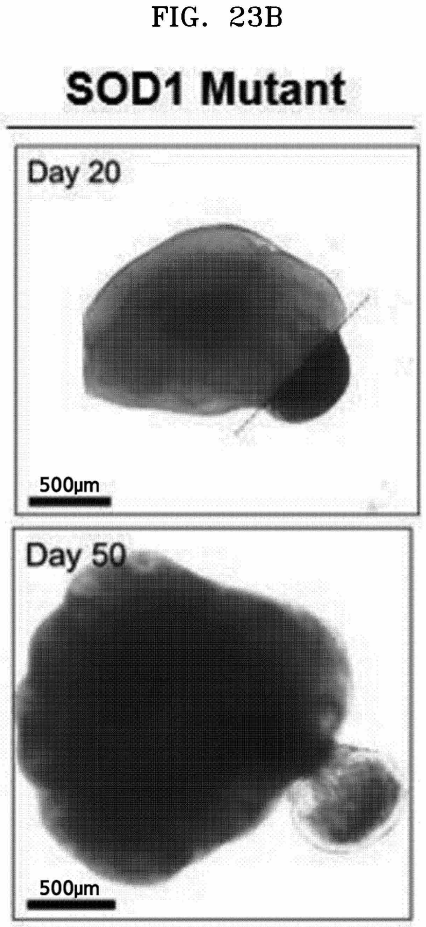

FIGS. 23A and 23B show representative morphologies of neuromuscular organoids generated from human induced pluripotent stem cells (hiPSCs) derived from amyotrophic lateral sclerosis (ALS) patients. Specifically, the representative morphologies of a wild-type neuromuscular organoid (FIG. 23A) and a neuromuscular organoid generated from hiPSCs derived from ALS patients (FIG. 23B) are shown.

### Best Mode for Carrying out the Invention

The inventors of the present disclosure developed neuromuscular organoids through three-dimensional culture of human pluripotent (totipotent) stem cells, thereby completing the present disclosure.

The present disclosure provides a medium composition for culturing a neuromuscular organoid, for three-dimensional culture of pluripotent stem cells (PSCs) into a neuromuscular organoid, the medium composition including a sonic hedgehog (SHH) signaling activator as an active ingredient.

In the present disclosure, "for culture" refers to "usable in any culture process", and may refer to, for example, "for use in culture" and "useful for culture". Therefore, "for culture" may be used interchangeable with "for use in culture" and "useful for culture", but the embodiment is not limited thereto.

In the present disclosure, sonic hedgehog (SHH) may be expressed from notochord, which temporarily exists beneath the spinal cord before the spine is formed during development, and neural stem cells in the spinal cord, located on the ventral side near the notochord, may exhibit an effect that promotes differentiation into the floor plate and motor neurons according to the concentration gradient of the SHH, but the embodiment is not limited thereto.

In the present disclosure, "SHH" refers to a protein that is one of three or more proteins in the mammalian signaling pathway family called hedgehog, another of these proteins is desert hedgehog (DHH) and yet another of these proteins is Indian hedgehog (IHH), but the embodiment is not limited thereto. SHH interacts with two or more transmembrane proteins through interactions with transmembrane molecules- Patched (PTC) and Smoothened (SMO). Typically, SHH binds to PTC and acts as a signal transducer, allowing activation of SMO, and in the absence of SHH, PTC typically suppresses SMO, which in turn activates transcriptional repressors to prevent transcription of specific genes, but the embodiment is not limited thereto. Furthermore, when SHH is present and binds to PTC, PTC cannot interfere with the function of SMO. When SMO is not suppressed, certain proteins may act as transcription factors that allow certain proteins to enter the nucleus and activate certain genes, but the embodiment is not limited thereto.

In the present disclosure, SHH signaling may be associated with differentiation of neural stem cells into motor neurons, such as lower motor neurons (LMNs). Here, LMNs form neuromuscular junctions with skeletal muscles, and may be formed on the ventral side of the spinal cord, but the embodiment is not limited thereto.

In the present disclosure, "SHH signaling activator" may refer to any molecule or compound that activates the SHH signaling pathway, including molecules or compounds that bind to a PTC or SMO agonist or the like. However, the embodiment is not limited thereto.

In the present disclosure, "signal" may refer to internal and external factors that regulate changes in the structure and function of cells, and may include both chemical and physical factors. However, the embodiment is not limited thereto.

In the present disclosure, the term "activator" or "activation" may refer to any molecule, such as a small molecule, a peptide, a protein, or a compound, for activating a molecule that induces directed differentiation of cells of the present disclosure, but the embodiment is not limited thereto.

In the present disclosure, "active ingredient" refers to an ingredient that exhibits the desired activity either alone or in combination with a carrier or the like that is not itself active.

In the present disclosure, "organoid" may refer to three-dimensional cell aggregates formed through self-replication and self-organization derived from pluripotent stem cells (PSCs), adult stem cells (ASCs), embryonic stem cells (ESCs), induced pluripotent stem cells (iPSCs), etc., and may include an organoid or cell cluster formed using suspension cell cultures. The organoid may also be referred to as a miniature organ, an organ-like structure, or a pseudo-organ. The organoid specifically includes one or more cell types among various types of cells that constitute organs or tissue, and may reproduce the shape and function of organs or tissue. Furthermore, by re-aggregating and recombining cells using three-dimensional culture methods to mimic the living environment, the limitations of two-dimensional (2D) cell lines cultured using 2D culture methods are overcome, enabling the faithful reproduction of physiological functions in living organisms, and thus applications to disease modeling, drug screening, and the like may be achieved. However, the embodiment is not limited thereto.

In the present disclosure, "stem cells" may refer to undifferentiated cells having self-renewal ability and differentiation proliferation ability. The stem cells may be subdivided into sub-groups based on the differentiation ability, including pluripotent stem cells, multipotent stem cells, bipotent stem cells, and the like. The pluripotent stem cells may refer to cells having the ability to differentiate into all tissues or cells that constitute a living body. The multipotent stem cells may refer to cells having the ability to differentiate into multiple, but not all, types of tissues or cells. The unipotent stem cells may refer to cells having the ability to differentiate into a specific tissue or cell. The pluripotent stem cells may include embryonic stem cells (ES cells), undifferentiated embryonic germ cells (EG cells), dedifferentiated stem cells (e.g., induced pluripotent stem cells, iPSCs), and the like. The multipotent stem cells may include somatic stem cells such as mesenchymal cells (derived from adipose, bone marrow, cord blood or umbilical cord, etc.), hematopoietic stem cells (derived from bone marrow or peripheral blood, etc.), neural stem cells, germline stem cells, and the like. However, the embodiment is not limited thereto.

In the present disclosure, "PSCs" may be used interchangeably with totipotent stem cells, but the embodiment is not limited thereto.

In the present disclosure, "pluripotency or pluripotent" may refer to the ability to develop into any of three germ layers: endoderm (e.g., gastric inner layer, gastrointestinal tract, lungs), mesoderm (e.g., muscle, bone, blood, urogenital tract), or ectoderm (e.g., epidermal tissue and nervous system), but the embodiment is not limited thereto.

In the present disclosure "iPSCs" refer to cells induced by artificially performing a dedifferentiation process (reprogramming) on fully differentiated somatic cells, and these cells may have pluripotency. The iPSCs may differentiate into cells of various organs such as the brain and heart, but the embodiment is not limited thereto. Furthermore, in the present disclosure, "iPSCs" may refer to iPSCs derived from fibroblasts, peripheral blood mononuclear cells, skin keratinocytes, neurons, or umbilical cord blood, but the embodiment is not limited thereto. In an embodiment of the present disclosure, the iPSCs may be fibroblast-derived iPSCs, but the embodiment is not limited thereto.

In the present disclosure, "somatic cells" are opposite to embryonic cells, referring to cells derived from an adult that is born and survives. In an embodiment of the present disclosure, the somatic cells may be fibroblasts, peripheral blood mononuclear cells, skin keratinocytes, neurons, or umbilical cord blood. In the present disclosure, the somatic cells may be derived from fibroblasts, peripheral blood mononuclear cells, skin keratinocytes, neurons, or umbilical cord blood, but the embodiment is not limited thereto.

In the present disclosure "neuromuscular organoid" may be formed by co-development of nervous tissue, skeletal muscle tissue, and a neuromuscular junction from the same precursor cells, but the embodiment is not limited thereto. Furthermore, in the present disclosure, the neuromuscular junction may refer to synaptic connection between the terminal of a motor nerve and a muscle (e.g., skeletal, smooth, or cardiac), but the embodiment is not limited thereto.

In the present disclosure "neuromuscular organoid" may differ from a neuromuscular organoid according to the prior art, which lacks the notochord expressing SHH in the embryo and lacks the SHH signaling essential for differentiation into motor neurons. However, the embodiment is not limited thereto.

In the present disclosure, "neuromuscular organoid" may be used for studying the mechanisms of neurological and muscular disease and used as a drug screening model, by reproducing nervous tissue, muscle tissue, and a junction between these two tissues in vitro from human-derived cells. However, the embodiment is not limited thereto.

In the present disclosure, "neuromuscular organoid" refers to one in which differentiation into motor neurons has been successfully achieved, and may be used as a model for studies on neuromuscular diseases (e.g., degenerative neuromuscular diseases) and for development of therapeutic agents. However, the embodiment is not limited thereto.

In the present disclosure, "neuromuscular organoid" may exhibit synchronous contraction not observed in a neuromuscular organoid when applying conventional techniques, and such synchronous contraction may be remarkably similar to the contraction of skeletal muscle tissue. However, the embodiment is not limited thereto.

In the present disclosure, "synchronous contraction" may be used interchangeably with synchronized contraction and simultaneous contraction, but the embodiment is not limited thereto.

In the present disclosure, synchronous contraction may refer to much large contraction of the entire skeletal muscle of the neuromuscular organoid, as opposed to spasm of skeletal muscle, but the embodiment is not limited thereto.

In the present disclosure, "synchronous contraction" may be caused by proliferation and maturation of skeletal muscle cells, but the embodiment is not limited thereto.

In the present disclosure, "neuromuscular disease" may refer to a broad range of diseases involving damage or dysfunction of peripheral nerves or muscles. Specifically damaged sites may be the cell body, axon, Schwann cells, neuromuscular junction, muscle, or a combination thereof, but the embodiment is not limited thereto.

In the present disclosure, "neuromuscular disease" may be a degenerative neuromuscular disease. Specifically, in the present disclosure, the degenerative disease may be spinal muscular atrophy (SMA), amyotrophic lateral sclerosis (ALS), Duchenne muscular dystrophy, and myotonic dystrophy, but the embodiment is not limited thereto.

In the present disclosure, "culture" may refer to any act performed to grow cells under appropriately artificially controlled environmental conditions, but the embodiment is not limited thereto.

In an embodiment of the present disclosure, culture may include 2D culture or 3D culture, but the embodiment is not limited thereto.

In the present disclosure, an SHH signaling activator may be at least one selected from the group consisting of an SHH signaling agonist and an SHH protein, but the embodiment is not limited thereto.

In the present disclosure, the SHH signaling agonist may be at least one selected from the group consisting of purmorphamine and a smoothened agonist (SAG), but the embodiment is not limited thereto.

In the present disclosure, "purmorphamine" refers to a purine derivative, having CAS No. 483367-10-8. For example, it may activate the Hedgehog pathway including targeting Smoothened, and may be a compound of [Formula 1], but the embodiment is not limited thereto:

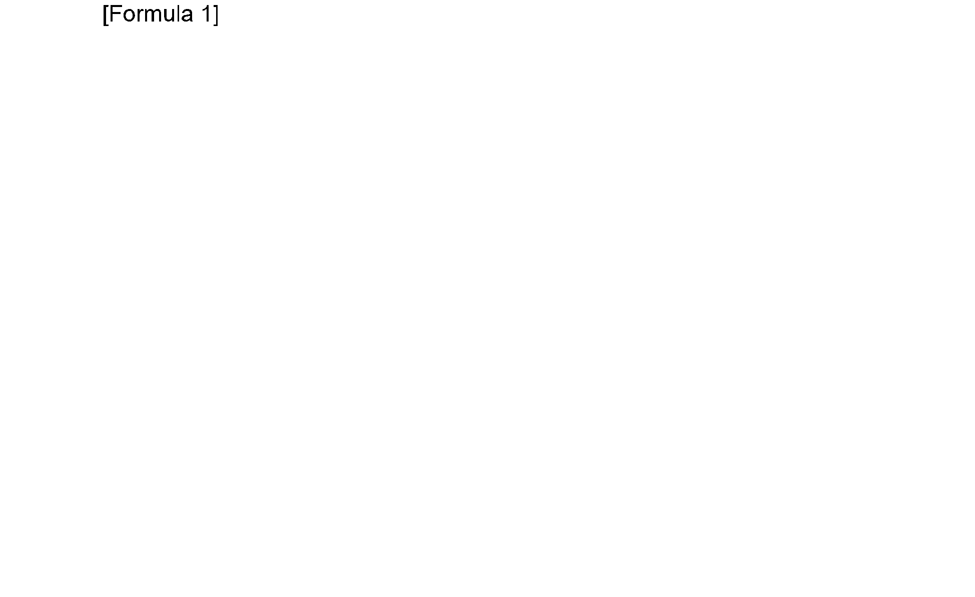

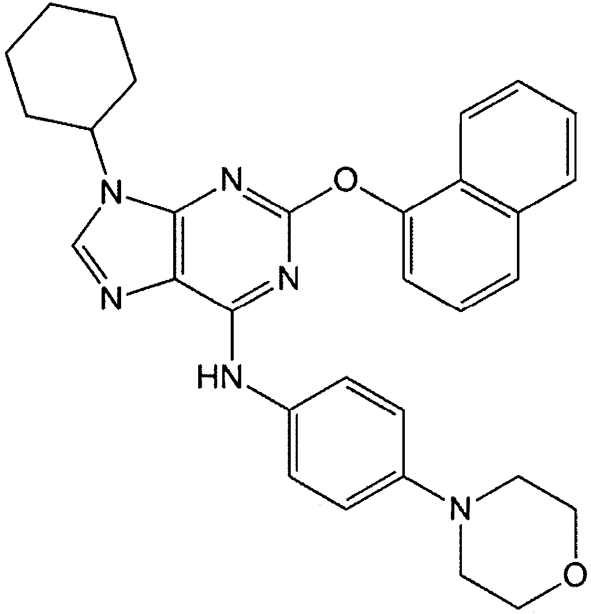

In a detailed embodiment of the present disclosure, purmorphamine may be Stemolecule^{™} purmorphamine (Stemgent, Inc. Cambridge, Massachusetts, United States), or otherwise, may be obtained and used either by direct manufacture or through commercially available sources. However, the embodiment is not limited thereto.

The purmorphamine of the present disclosure serves as an SHH signaling agonist, and may promote differentiation of neural stem cells into motor neurons. The purmorphamine of the present disclosure may activate the SHH signaling during the formation of a neuromuscular organoid, but the embodiment is not limited thereto.

"Smoothened agonist (SAG)" of the present disclosure may be a chlorobenzothiophene-containing compound, having CAS No: 364590-63-6, that acts as an activator of G protein-bound receptor, Smoothened (SMO). Otherwise, it may be obtained and used by direct synthesis or commercially available sources, and may be a compound of [Formula 2], but the embodiment is not limited thereto:

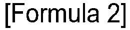

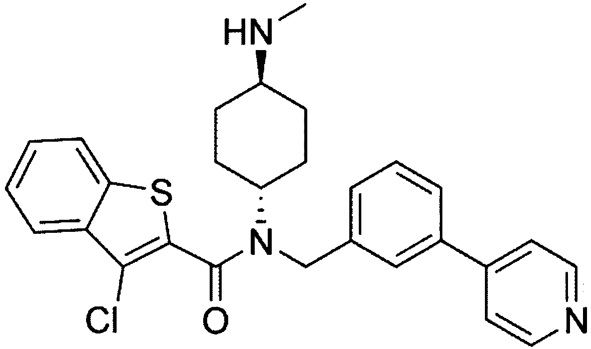

In the present disclosure, a medium composition may further contain at least one selected from the group consisting of glycogen synthase kinase 3 (GSK-3) inhibitor, fibroblast growth factor (FGF), Y-27632, insulin-like growth factor (IGF), and hepatocyte growth factor (HGF), but the embodiment is not limited thereto.

In the present disclosure, "GSK-3 inhibitor" may be an inhibitor of serine/threonine protein kinases that mediate addition of phosphate molecules to serine residues and threonine residues, but the embodiment is not limited thereto. Furthermore, in the present disclosure, "GSK-3 inhibitor" may be, for example, CHIR99021, but the embodiment is not limited thereto.

In the present disclosure, "CHIR99021" is 6-[[2-[[4-(2,4-Dichlorophenyl)-5-(5-methyl-1H-imidazol-2-yl)-2-pyrimidinyl]amino]ethyl]amino]-3-pyridinecarbonitrile, and may be a substance serving as a GSK inhibitor targeting upstream molecules of GSK1/2 involved in the GSK signaling pathway, but the embodiment is not limited thereto. CHIR99021 may be used at a concentration in a range of 2 to 4 µM or 2.5 to 3.5 µM. More specifically, CHIR99021 may be used at a concentration of 3 µM, but the embodiment is not limited thereto.

In the present disclosure, "fibroblast growth factor (FGF)" may be a secretory molecule that plays a role in proliferation, differentiation, and recovery after damage of stem cells, but the embodiment is not limited thereto. Furthermore, in the present disclosure, "FGF" may be, for example, basic fibroblast growth factor (bFGF), but the embodiment is not limited thereto.

In the present disclosure, "bFGF" refers to basic FGF, which may promote proliferation of various cells or induce differentiation of various cells, and may exhibit activity by binding to bFGF receptors on the cell surface, but the embodiment is not limited thereto.

In the present disclosure, bFGF may be a factor involved in angiogenesis, wound healing, embryonic development, and various endocrine signaling pathways. bFGF may play a crucial role in proliferation and differentiation processes of various cells and tissues, but the embodiment is not limited thereto. bFGF may be used at a concentration in a range of 7 to 12 ng/mL or 8 to 11 ng/mL. More specifically, bFGF may be used at a concentration of 10 ng/mL, but the embodiment is not limited thereto.

In the present disclosure, Y-27632 may be used at a concentration in a range of 40 to 60 µM or 45 to 55 µM, and more specifically, at a concentration of 50 µM, but the embodiment is not limited thereto.

In the present disclosure, "IGF" may be a factor secreted primarily in the liver and intestines in response to stimulation by growth hormone (GH). Most cells in the human body (particularly, cells in muscles, bones, liver, kidneys, nerves, skin, and lungs) may be influenced by IGF1, and may have functions that regulate cell growth (especially of neurons) and development, as well as DNA synthesis in cells, in addition to insulin-like effects. However, the embodiment is not limited thereto. Furthermore, in the present disclosure, "IGF" may be insulin-like growth factor-1 (IGF-1), but the embodiment is not limited thereto. IGF-1 may be used at a concentration in a range of 1 to 3 ng/mL or 1.5 to 2.5 ng/mL, and more specifically, at a concentration of 2 ng/mL, but the embodiment is not limited thereto.

In the present disclosure, HGF may be a paracrine cell growth factor, a motility factor, and a morphogenesis factor, secreted by mesenchymal cells, primarily acting on epithelial and endothelial cells, but may also act on hematopoietic progenitor cells and T cells. However, the embodiment is not limited thereto. HGF may be used at a concentration in a range of 1 to 3 ng/mL or 1.5 to 2.5 ng/mL, and more specifically, at a concentration of 2 ng/mL, but the embodiment is not limited thereto.

In the present disclosure, "medium" may refer to a medium capable of supporting proliferation, survival, and differentiation of a neuromuscular organoid *in vitro.* All conventional media suitable for culture and differentiation of neuromuscular organoids used in the relevant field may be used, but the embodiment is not limited thereto. Furthermore, the medium type and culture conditions may be appropriately selected according to the cell types, but the embodiment is not limited thereto.

In the present disclosure, at least one selected from the group consisting of GSK-3 inhibitor, FGF, Y-27632, IGF, and HGF may be added to a basic medium to exhibit an effect, but the embodiment is not limited thereto.

In the present disclosure, the basic medium may be any basic medium commonly used in the art, but the embodiment is not limited thereto.

In an embodiment of the present disclosure, the basic medium may be a stabilized feeder-free maintenance medium for human pluripotent stem cells (hPSCs), but the embodiment is not limited thereto. Specifically, mTeSR1 plus medium or N2B27 medium may be used, but the embodiment is not limited thereto.

In the present disclosure, mTeSR1 plus medium (catalog No: 100-1130 or 100-0276) may be used, but the embodiment is not limited thereto.)mTeSR1 plus medium (by Stem Cell Technologies)may be used, which may maintain cell quality characteristics and enhance cell proliferation rates through stabilized key medium components including FGF2 and enhanced pH buffering function, but the embodiment is not limited thereto.

In the present disclosure, N2B27 medium may be a mixture of DMEM/F12 supplemented with 1 x N2 and neurobasal medium supplemented with 1 x B27, 1 x Glutamax, 0.1 mM beta-mercaptoethanol (Simga), and 1 x Pen-Strep antibiotic, but the embodiment is not limited thereto.

In the present disclosure, N2B27 medium may be a mixture of DMEM/F12 supplemented with 1 x N2 and neurobasal medium supplemented with 1 x B27, 1 x Glutamax, 0.1 mM beta-mercaptoethanol (Simga), 1 x Pen-Strep antibiotic, mixed at a ratio of 1:10 to 10:1, 2:10 to 10:2, 3:10 to 10:3, 4:10 to 10:4, 6:10 to 10:6, 8:10 to 10:8, 9:10 to 10:9, or 1:1, but the embodiment is not limited thereto.

In the present disclosure, 1 x N2 (of which the catalog number is, although not limited thereto, 17502048 or 17502001) may be 1 x N2 (Gibco), but the embodiment is not limited thereto.

In the present disclosure, DMEM/F12 (of which the catalog number is, although not limited thereto, 11320033 or 11320082)may be DMEM/F12 (Gibco), which is a basic medium used to assist the growth of mammalian cells. Cells successfully cultured in DMEM/F12 may include MDCK, glial cells, fibroblasts, human endothelial cells, and mouse fibroblasts, but the embodiment is not limited thereto.

In the present disclosure, 1 x B27 (of which the catalog number is, although not limited thereto, 17504044 or 17504001) may be1 x B27 (Gibco), but the embodiment is not limited thereto.

In the present disclosure, 1 x Glutamax (of which the catalog number is, although not limited thereto, 35050061 or 35050079) may be1 x Glutamax (Gibco), but the embodiment is not limited thereto.

In the present disclosure, 1 x Pen-Strep antibiotic (of which the catalog number is, although not limited thereto, LS 202-01 or LS 202-02) may be1 x Pen-Strep antibiotic (Welgene), but the embodiment is not limited thereto.

In the present disclosure, neurobasal medium (of which the catalog number is, although not limited thereto, 21103049) may beneurobasal medium (Gibco). When used with Gibco B-27 supplement, the medium may be a basic medium designed for the long-term maintenance and maturation of pure prenatal and embryonic neuronal populations without the need for an astrocyte trophic layer, but the embodiment is not limited thereto.

In an embodiment of the present disclosure, "medium composition" may be a medium composition for culture, but the embodiment is not limited thereto.

In an embodiment of the present disclosure, medium composition may be individually treated by a combination of one or more components, but the embodiment is not limited thereto. For example, the medium composition of the present disclosure may be divided into three or more stages and individually designated as a first composition to a third composition.

In an embodiment of the present disclosure, the first composition may be a medium composition for differentiating subject-derived PSCs into NMPs, and may contain CHIR99021 and bFGF, but the embodiment is not limited thereto. In the present disclosure, a process of differentiating subject-derived PSCs into NMPs using the first composition may be termed a "pretreatment" process. In this regard, the first composition of the present disclosure may be used interchangeably with "pretreatment composition", but the embodiment is not limited thereto.

Furthermore, the second composition may be a medium composition for further diluting separated and treated NMPs to prevent intercellular binding, and may contain Y-27632, bFGF, IGF-1, and HGF, but the embodiment is not limited thereto. Furthermore, the second composition of the present disclosure may be used prior to the formation of embryoid bodies by the NMPs pretreated using the first composition, but the embodiment is not limited thereto.

Lastly, the third composition may be a medium composition for culturing an embryoid body into a neuromuscular organoid, and may contain an SHH signaling agonist. It may further contain one or more of the five components (i.e., CHIR99021, bFGF, Y-27632, IGF-1, and HGF), wherein the further contained components may be IGF-1 and HGF, but the embodiment is not limited thereto.

In an embodiment of the present disclosure, the medium composition may contain the five components (i.e., CHIR99021, bFGF, Y-27632, IGF-1, and HGF), wherein each component may be independently contained and processed in one or more stages, but the embodiment is not limited thereto.

In the present disclosure, the neuromuscular organoid may include one or more selected from the group consisting of nervous tissue, skeletal muscle tissue, and the neuromuscular junction.

In an embodiment of the present disclosure, when a neuromuscular organoid is produced using the medium composition of the present disclosure, the produced neuromuscular organoid was confirmed to include all of the nervous tissue, skeletal muscle tissue, and neuromuscular junction. According to common knowledge in the art, it is considerably difficult to produce a neuromuscular organoid including all such neuromuscular components. Nevertheless, the inventors of the present disclosure have demonstrated that the nervous tissue organoid resembling the human neuromuscular tissue could be produced, as evidenced by the fact that the neuromuscular organoid including all components of the nervous tissue, skeletal muscle tissue, and neuromuscular junction, rather than only some of these components, when using the medium composition of the present disclosure. Such results may suggest excellent effectiveness of the present disclosure.

In the present disclosure, the PSCs may be human induced pluripotent stem cells (hiPSCs), but the embodiment is not limited thereto.

In an embodiment of the present disclosure, the PSCs may be human embryonic stem cells (hESCs) or hiPSCs, but the embodiment is not limited thereto.

The present disclosure provides a manufacturing method for a neuromuscular organoid, the method including the following processes:
(S0) culturing human pluripotent cells (hPSCs) derived from a subject in a medium containing glycogen synthase kinase 3 (GSK-3) inhibitor and fibroblast growth factor (FGF) to differentiate into bipotent neuromesodermal progenitors (NMPs);
(S1) diluting the bipotent NMPs, followed by dispensing, to form embryoid bodies; and
(S2) culturing the embryoid body in a medium containing a sonic hedgehog (SHH) signaling activator to produce a three-dimensional neuromuscular organoid.

The manufacturing method for a neuromuscular organoid by the treatment with the SHH signaling activator of the present disclosure (specifically, although not limited thereto, purmorphamine) may involve activating the SHH signaling and successfully inducing differentiation into motor neurons without inhibiting the formation of a neuromuscular organoid, but the embodiment is not limited thereto.

In an embodiment of the present disclosure, processes (S0), (S1), and (S2) may each last from 1 day to 60 days, 1 day to 55 days, 1 day to 50 days, 1 day to 45 days, 1 day to 40 days, 1 day to 35 days, 1 day to 30 days, 1 day to 28 days, 1 day to 26 days, 1 day to 24 days, 1 day to 22 days, 1 day to 20 days, 1 day to 18 days, 1 day to 16 days, 1 day to 14 days, 1 day to 12 days, 1 day to 10 days, 1 day to 9 days, 1 day to 8 days, 1 day to 7 days, 1 day to 6 days, 1 day to 5 days, 1 day to 4 days, 1 day to 3 days, 1 day to 2 days, 2 days to 60 days, 2 days to 55 days, 2 days to 50 days, 2 days to 45 days, 2 days to 40 days, 2 days to 35 days, 2 days to 30 days, 2 days to 28 days, 2 days to 26 days, 2 days to 24 days, 2 days to 22 days, 2 days to 20 days, 2 days to 18 days, 2 days to 16 days, 2 days to 14 days, 2 days to 12 days, 2 days to 10 days, 2 days to 9 days, 2 days to 8 days, 2 days to 7 days, 2 days to 6 days, 2 days to 5 days, 2 days to 4 days, 2 days to 3 days, 3 days to 60 days, 3 days to 55 days, 3 days to 50 days, 3 days to 45 days, 3 days to 40 days, 3 days to 35 days, 3 days to 30 days, 3 days to 28 days, 3 days to 26 days, 3 days to 24 days, 3 days to 22 days, 3 days to 20 days, 3 days to 18 days, 3 days to 16 days, 3 days to 14 days, 3 days to 12 days, 3 days to 10 days, 3 days to 9 days, 3 days to 8 days, 3 days to 7 days, 3 days to 6 days, 3 days to 5 days, 3 days to 4 days, 5 days to 60 days, 5 days to 55 days, 5 days to 50 days, 5 days to 45 days, 5 days to 40 days, 5 days to 35 days, 5 days to 30 days, 5 days to 28 days, 5 days to 26 days, 5 days to 24 days, 5 days to 22 days, 5 days to 20 days, 5 days to 18 days, 5 days to 16 days, 5 days to 14 days, 5 days to 12 days, 5 days to 10 days, 5 days to 9 days, 5 days to 8 days, 5 days to 7 days, 5 days to 6 days, 10 days to 60 days, 10 days to 55 days, 10 days to 50 days, 10 days to 45 days, 10 days to 40 days, 10 days to 35 days, 10 days to 30 days, 10 days to 28 days, 10 days to 26 days, 10 days to 24 days, 10 days to 22 days, 10 days to 20 days, 10 days to 18 days, 10 days to 16 days, 10 days to 14 days, 10 days to 12 days, 15 days to 60 days, 15 days to 55 days, 15 days to 50 days, 15 days to 45 days, 15 days to 40 days, 15 days to 35 days, 15 days to 30 days, 15 days to 28 days, 15 days to 26 days, 15 days to 24 days, 15 days to 22 days, 15 days to 20 days, 15 days to 18 days, 15 days to 16 days, 20 days to 60 days, 20 days to 55 days, 20 days to 50 days, 20 days to 45 days, 20 days to 40 days, 20 days to 35 days, 20 days to 30 days, 20 days to 28 days, 20 days to 26 days, 20 days to 24 days, or 20 days to 22 days, but the embodiment is not limited thereto.

In an embodiment of the present disclosure, each of processes (S0), (S1), and (S2) may start from the point at which the embryoid body is formed as Day 0, but the embodiment is not limited thereto.

In an embodiment of the present disclosure, process (S0) may begin within -10 days to 0 days, -9 days to 0 days, -8 days to 0 days -7 days to 0 days, -6 days to 0 days, -5 days to 0 days, -4 days to 0 days, -3 days to 0 days, -2 days to 0 days, -1 day to 0 day, or on -5 days, -4 days, -3 days, -2 days, or -1 day, but the embodiment is not limited thereto.

In an embodiment of the present disclosure, process (S1) may begin on Day 0, but the embodiment is not limited thereto.

In an embodiment of the present disclosure, process (S2) may begin within 1 day to 15 days, 1 day to 14 days, 1 day to 13 days, 1 day to 12 days, 1 day to 11 day, 1 day to 10 days, 1 day to 9 days, 1 day to 8 days, 1 day to 7 days, 1 day to 6 days, 1 day to 5 days, 1 day to 4 days, 1 day to 3 days, 1 day to 2 days, 2 days to 15 days, 2 days to 14 days, 2 days to 13 days, 2 days to 12 days, 2 days to 11 day, 2 days to 10 days, 2 days to 9 days, 2 days to 8 days, 2 days to 7 days, 2 days to 6 days, 2 days to 5 days, 2 days to 4 days, 2 days to 3 days, 3 days to 15 days, 3 days to 14 days, 3 days to 13 days, 3 days to 12 days, 3 days to 11 day, 3 days to 10 days, 3 days to 9 days, 3 days to 8 days, 3 days to 7 days, 3 days to 6 days, 3 days to 5 days, 3 days to 4 days, 4 days to 15 days, 4 days to 14 days, 4 days to 13 days, 4 days to 12 days, 4 days to 11 day, 4 days to 10 days, 4 days to 9 days, 4 days to 8 days, 4 days to 7 days, 4 days to 6 days, or 4 days to 5 days, or on day 4, but the embodiment is not limited thereto.

The manufacturing method for a neuromuscular organoid of the present disclosure, unlike methods involving separating PSCs into single cell state, followed by differentiation, may allow differentiation of PSCs in a colony state, but the embodiment is not limited thereto.

The manufacturing method for a neuromuscular organoid of the present disclosure includes culturing in a medium containing an SHH signaling activator to produce a 3D neuromuscular organoid, wherein, the neuromuscular organoid is characterized by, although not limited thereto, at least one selected from the following group consisting of:
(a) increased expression of motor neuron markers;
(b) promoted proliferation of skeletal muscle cells;
(c) promoted maturation of skeletal muscle cells; and
(d) synchronous contraction.

The manufacturing method for a neuromuscular organoid of the present disclosure may include inducing differentiation of PSCs into NMPs, which are bipotent progenitors capable of differentiating into both neurons and muscle cells that constitute the posterior spinal cord, but the embodiment is not limited thereto.

The PSCs of the present disclosure capable of undergoing differentiation while being in a colony state may exhibit an effect of inducing excellent formation and differentiation of embryoid bodies, but the embodiment is not limited thereto.

In the present disclosure, "bipotent NMPs" contribute to both the spinal cord and the paraxial mesoderm and are capable of differentiating into multiple cell types, and specifically, may be differentiated into both neurons and skeletal muscle cells, but the embodiment is not limited thereto.

The NMPs of the present disclosure, unlike PSCs, have limited differentiation ability. In this regard, differentiation into cell types other than neurons and somatic mesoderm-derived cells may be restricted, but the embodiment is not limited thereto.

In the present disclosure, "subject" is not limited unless it is vertebrates. Specifically, the subject may be a human, a mouse, a rat, a guinea pig, a rabbit, a monkey, a pig, a horse, a cow, sheep, a goat, a dog, and a cat. In an embodiment of the present disclosure, the subject may be a human, but the embodiment is not limited thereto.

In an embodiment of the present disclosure, the subject may be a normal subject or a neuromuscular disease subject, but the embodiment is not limited thereto. Furthermore, it was confirmed in the present disclosure that, when PSCs derived from a normal subject are used, normal neuromuscular organoids could be produced, whereas, when PSCs derived from a neuromuscular disease subject are used, neuromuscular organoids of the neuromuscular disease subject could be produced.

In the present disclosure, "dilution" may be for the purpose of preventing intercellular binding, and may involve adjusting the cell concentration in a medium to ensure that a certain level of cells is included.

In a specific embodiment of the present disclosure, dilution is intended to adjust the cell concentration in a medium to contain 2,000 cells, but the embodiment is not limited thereto.

In the present disclosure, "differentiation" may refer to a process whereby unspecialized cells acquire the characteristics of specialized cells, such as certain types of neurons, muscle cells, brain cells, heart cells, or liver cells, but the embodiment is not limited thereto. Furthermore, "differentiation" may be regulated by interactions of genes in a cell and under physical and chemical conditions outside a cell via signaling pathways involving proteins generally present on the cell surface, but the embodiment is not limited thereto.

In the present disclosure, the unspecified cells may include PSCs, bipotent NMPs, embryoid bodies, etc., but the embodiment is not limited thereto.

In the present disclosure, "dispensing" may involve diluting and dispensing the bipotent NMPs of the present disclosure to form embryoid bodies, but the embodiment is not limited thereto.

In the present disclosure, in process (S0), the hPSCs may be in a colony state, but the embodiment is not limited thereto.

In the present disclosure, "colony state" may refer to a state not including a process of treating PSCs with a dissociating agent, such as Accutase, to be separated into individual cells, but the embodiment is not limited thereto.

In the present disclosure, "colony state" may exhibit superior effects of inducing formation of or differentiation into embryoid bodies, compared to the case where cells that have been separated into individual cells are cultured to differentiate into bipotent NMPs, but the embodiment is not limited thereto.

In the present disclosure, process (S0) may be used interchangeably with a "pretreatment" process, but the embodiment is not limited thereto.

In the present disclosure, in process (S0), the bipotent NMPs may express SRY sex-determining region Y-box 2 (SOX2) or BRACHYURY (BRA), but the embodiment is not limited thereto.

In the present disclosure, in process (S0), the differentiated bipotent NMPs may express SOX2 or BRA, but the embodiment is not limited thereto.

In process (S1) of the present disclosure, a medium composition for diluting the bipotent NMPs may contain at least one selected from the group consisting of Y-27632, FGF, IGF, and hepatocyte growth factor (HGF), but the embodiment is not limited thereto.

In process (S1) of the present disclosure, the medium composition for diluting the bipotent NMPs may contain at least one selected from the group consisting of Y-27632, FGF, IGF, and hepatocyte growth factor (HGF), but the embodiment is not limited thereto.

In the present disclosure, process (S1) may be, although not limited thereto, characterized by at least one selected from the following group consisting of:
(a) performing process (S1) before the bipotent NMPs form the embryoid body; and
(b) dispensing 1500 to 9000 bipotent NMPs.

In the present disclosure, process (S1) may be performed for 0 day to 6 days, but the embodiment is not limited thereto.

In process (S1) of the present disclosure, 0 day may refer to the period immediately following the completion of process (S0) or the subsequent period following process (S0), but the embodiment is not limited thereto. Furthermore, in process (S1) of the present disclosure, 0 day may refer to the period immediately following the pretreatment process or the subsequent period following the pretreatment process, but the embodiment is not limited thereto.

In the present disclosure, process (S1) may be performed before or during the formation of embryoid bodies from the bipotent NMPs, but the embodiment is not limited thereto.

In an embodiment of the present disclosure, in process (S1), the number of bipotent NMPs being dispensed may be in a range of 500 to 10000, 500 to 9500, 500 to 9000, 500 to 8500, 500 to 8000, 500 to 7500, 500 to 7000, 500 to 6500, 500 to 6000, 500 to 5500, 500 to 5000, 500 to 4500, 500 to 4000, 500 to 3500, 500 to 3000, 500 to 2500, 500 to 2000, 1000 to 10000, 1000 to 9500, 1000 to 9000, 1000 to 8500, 1000 to 8000, 1000 to 7500, 1000 to 7000, 1000 to 6500, 1000 to 6000, 1000 to 5500, 1000 to 5000, 1000 to 4500, 1000 to 4000, 1000 to 3500, 1000 to 3000, 1000 to 2500, 1000 to 2000, 1500 to 10000, 1500 to 9500, 1500 to 9000, 1500 to 8500, 1500 to 8000, 1500 to 7500, 1500 to 7000, 1500 to 6500, 1500 to 6000, 1500 to 5500, 1500 to 5000, 1500 to 4500, 1500 to 4000, 1500 to 3500, 1500 to 3000, 1500 to 2500, or 1500 to 2000, or may be 2000, the embodiment is not limited thereto. In an embodiment of the present disclosure, it was confirmed that neuromuscular organoids having excellent morphological characteristics were produced by dispensing 2000 bipotent NMPs.

In an embodiment of the present disclosure, process (S1) was performed for 4 days, but the embodiment is not limited thereto.

In the present disclosure, the culturing in process (S2) may be orbital shaking culture, but the embodiment is not limited thereto

In the present disclosure, process (S2) may begin within 1 day to 10 days, 1 day to 9 days, 1 day to 8 days, 1 day to 7 days, 1 day to 6 days, 1 day to 5 days, 1 day to 4 days, 1 day to 3 days, 1 day to 2 days, 2 days to 10 days, 2 days to 9 days, 2 days to 8 days, 2 days to 7 days, 2 days to 6 days, 2 days to 5 days, 2 days to 4 days, 2 days to 3 days, 3 days to 10 days, 3 days to 9 days, 3 days to 8 days, 3 days to 7 days, 3 days to 6 days, 3 days to 5 days, 3 days to 4 days, 4 days to 10 days, 4 days to 9 days, 4 days to 8 days, 4 days to 7 days, 4 days to 6 days, 4 days to 5 days, 5 days to 10 days, 5 days to 9 days, 5 days to 8 days, 5 days to 7 days, 5 days to 6 days, 6 days to 10 days, 6 days to 9 days, 6 days to 8 days, 6 days to 7 days, 7 days to 10 days, 7 days to 9 days, 7 days to 8 days, 8 days to 10 days, 8 days to 9 days, and 9 days to 10 days, starting from the completion time of process (S0), but the embodiment is not limited thereto.

In the present disclosure, process (S2) may begin within 1 day to 30 days, 1 day to 28 days, 1 day to 26 days, 1 day to 24 days, 1 day to 22 days, 1 day to 20 days, 1 day to 18 days, 1 day to 16 days, 1 day to 14 days, 1 day to 12 days, 1 day to 10 days, 1 day to 8 days, 1 day to 6 days, 1 day to 4 days, 1 day to 2 days, 2 days to 30 days, 2 days to 28 days, 2 days to 26 days, 2 days to 24 days, 2 days to 22 days, 2 days to 20 days, 2 days to 18 days, 2 days to 16 days, 2 days to 14 days, 2 days to 12 days, 2 days to 10 days, 2 days to 8 days, 2 days to 6 days, 2 days to 4 days, 3 days to 30 days, 4 days to 28 days, 4 days to 26 days, 4 days to 24 days, 4 days to 22 days, 4 days to 20 days, 4 days to 18 days, 4 days to 16 days, 4 days to 14 days, 4 days to 12 days, 4 days to 10 days, 4 days to 8 days, and 4 days to 6 days, starting from the completion time of process (S0), but the embodiment is not limited thereto.

In an embodiment of the present disclosure, process (S2) was performed for 4 days or up to 6 days, 10 days, 14 days, or 20 days, from the day of culture, and in other words, it was performed for a minimum period of about at least one or two days, but the embodiment is not limited thereto.

In the present disclosure, the orbital shaking culture may include rotary orbital shaking culture, but the embodiment is not limited thereto.

The present disclosure provides a 3D neuromuscular organoid produced by the manufacturing method of the present disclosure.

In the present disclosure, the organoid may express at least one selected from the group consisting of neuron-specific class **III** beta-tubulin (TUJ1), fast myosin heavy chain (MHC), SOX2, SHH, α-bungarotoxin (α-BTX), myosin heavy chain 2 (MYH2), and myosin heavy chain 7 (MYH7), but the embodiment is not limited thereto.

In the present disclosure, the neuromuscular organoid may be, although not limited thereto, characterized by at least one selected from the following group consisting of:
(a) decreased expression of Cdo and Tubb3; and
(b) increased expression of olig2 and choline acetyltransferase (ChAT).

In the present disclosure, the neuromuscular organoid may be, although not limited thereto, characterized by at least one selected from the following group consisting of:
(a) promoting differentiation into motor neurons;
(b) increasing the size of muscle fibers; and
(c) maturing muscle fibers.

In the present disclosure, the neuromuscular organoid may be, although not limited thereto, characterized by at least one selected from the following group consisting of:
(a) promoting differentiation into motor neurons;
(b) increasing the size of muscle fibers; and
(c) maturing muscle fibers.

In the present disclosure, 'the neuromuscular organoid characterized by promoting differentiation into motor neurons' may refer that, compared to conventional neuromuscular organoids, the neuromuscular organoid of the present disclosure significantly promotes differentiation into motor neurons, resulting in an increase in the number of cells being differentiated into motor neurons, but the embodiment is not limited thereto.

In the present disclosure, the neuromuscular organoid may be for differentiation into motor neurons, but the embodiment is not limited thereto. According to common knowledge in the art, it is known that, upon application of conventional technical knowledge, only 6 % of the neuromuscular organoid achieved differentiation into motor neurons. However, the inventors of the present disclosure confirmed that the neuromuscular organoid achieved a significantly excellent level of motor neuron differentiation efficiency, when treated with purmorphamine.

In the present disclosure, the increasing of the size of muscle fibers in the neuromuscular organoid may refer that skeletal muscle tissue becomes large, specifically refer that nervous tissue becomes small and skeletal muscle tissue becomes large, and more specifically refer that the growth rate of skeletal muscle tissue is relatively higher than that of nervous tissue. However, the embodiment is not limited thereto.

In the present disclosure, the increasing of the size of muscle fibers in the neuromuscular organoid may refer that treatment with the SHH signaling agonist causes nervous tissue to become small and skeletal muscle tissue to become large, and may imply that the increase in the overall size of the organoid is primarily due to the growth of skeletal muscle tissue rather than nervous tissue. However, the embodiment is not limited thereto.

In the present disclosure, the maturating of muscle fibers in the neuromuscular organoid may be confirmed by increased expression of mature muscle fiber markers, indicating early expression of mature muscle fiber markers, and may be also confirmed by synchronous contraction exhibited when muscle fibers mature. However, embodiment is not limited thereto.

In the present disclosure, the neuromuscular organoid may be characterized by synchronous contraction, but the embodiment is not limited thereto.

The present disclosure provides a neuromuscular disease model including the neuromuscular organoid.

The neuromuscular disease model overcomes the disadvantages of conventional nervous tissue, skeletal muscle tissue, and neuromuscular junction models (specifically animal models). It not only has a relatively simple manufacturing process, but also enables large-scale drug screening, and thus may be suitable as a platform for the prevention of neuromuscular diseases and/or development of therapeutic agents for neuromuscular diseases. However, the embodiment is not limited thereto.

In the present disclosure, the neuromuscular disease may be a degenerative neuromuscular disease, but the embodiment is not limited thereto.

In the present disclosure, the degenerative neuromuscular disease may be selected from the group consisting of SMA, ALS, Duchenne muscular dystrophy, and myotonic dystrophy, but the embodiment is not limited thereto.

In the present disclosure, degeneration or apoptosis of motor neurons may lead to weakening of the neuromuscular junctions and atrophy of muscle tissue, and conversely, muscular atrophy may cause weakening of the neuromuscular junctions and apoptosis of motor neurons. However, the embodiment is not limited thereto.

The present disclosure provides a screening method for a therapeutic agent for a neuromuscular disease, the method including the following processes:
(1) treating a candidate substance for treatment of a neuromuscular disease with the neuromuscular organoid derived from a neuromuscular disease subject manufactured by the manufacturing method of the present disclosure; and
(2) comparing morphological characteristics of the neuromuscular organoid treated with the candidate substance.

In the present disclosure, "screening method for a therapeutic agent for a neuromuscular disease" may be used interchangeably with "a screening method for a therapeutic agent for a neuromuscular disease," "method for use in screening for a therapeutic agent for a neuromuscular disease," and "method useful for screening for a therapeutic agent for a neuromuscular disease," but the embodiment is not limited thereto.

In the present disclosure, "candidate substance for treatment" may be an individual nucleic acid, a protein, other extracts or natural products, or a compound, randomly selected or presumed to have the potential for prevention or treatment of diseases related to neuromuscular diseases according to conventional selection methods. However, the embodiment is not limited thereto.

In the present disclosure, "treatment" may refer to any act whereby symptoms of a neuromuscular disease are ameliorated or beneficially altered by administration of a candidate substance for the treatment, but the embodiment is not limited thereto.

In the present disclosure, "neuromuscular organoid derived from a neuromuscular disease subject" may have the morphological characteristics of forming small muscle tissue (specifically, although not limited thereto, skeletal muscle tissue) compared to normal neuromuscular tissue, but the embodiment is not limited thereto.

To confirm whether abnormal morphological characteristics of the neuromuscular organoid derived from a neuromuscular disease subject of the present disclosure are simply due to batch-to-batch variability and mutant genotypes, repetition and additional characterization of the neuromuscular organoid derived from a neuromuscular disease subject may be required. However, embodiment is not limited thereto.

The size of muscle tissue formed in the neuromuscular organoid derived from a neuromuscular disease substance of the present disclosure may be 0.1 time to 0.9 times, 0.1 times to 0.8 times, 0.1 times to 0.7 times, 0.1 times to 0.6 times, 0.1 times to 0.5 times, 0.1 times to 0.4 times, 0.1 times to 0.3 times, 0.1 times to 0.2 times, 0.2 times to 0.9 times, 0.2 times to 0.8 times, 0.2 times to 0.7 times, 0.2 times to 0.6 times, 0.2 times to 0.5 times, 0.2 times to 0.4 times, 0.2 times to 0.3 times, 0.3 times to 0.9 times, 0.3 times to 0.8 times, 0.3 times to 0.7 times, 0.3 times to 0.6 times, 0.3 times to 0.5 times, 0.3 times to 0.4 times, 0.4 times to 0.9 times, 0.4 times to 0.8 times, 0.4 times to 0.7 times, 0.4 times to 0.6 times, 0.4 times to 0.5 times, 0.5 times to 0.9 times, 0.5 times to 0.8 times, 0.5 times to 0.7 times, 0.5 times to 0.6 times, 0.6 times to 0.9 times, 0.6 times to 0.8 times, 0.6 times to 0.7 times, 0.7 times to 0.9 times, 0.7 times to 0.8 times, and 0.8 times to 0.9 times the size of muscle tissue formed in normal neuromuscular tissue, but the embodiment is not limited thereto.

The screening method of the present disclosure may further include (3) determining the candidate substance as a therapeutic agent for a neuromuscular disease, when the morphological characteristics of the neuromuscular organoid treated with the candidate substance exhibit morphological characteristics of normal neuromuscular tissue, but the embodiment is not limited thereto.

In the present disclosure, the morphological characteristics of normal neuromuscular tissue may include formation of larger muscle tissue compared to the neuromuscular organoid derived from a neuromuscular disease subject, but the embodiment is not limited thereto.

In addition, the present disclosure provides use of a medium composition for three-dimensional culture of a neuromuscular organoid from PSCs, the medium composition including an SHH signaling activator as an active ingredient.

In addition, the present disclosure provides use of a composition for three-dimensional culture of a neuromuscular organoid from PSCs, the composition including an SHH signaling activator as an active ingredient.

In addition, the present disclosure provides use of a medium composition for manufacturing a neuromuscular organoid from PSCs, the composition including as SHH signaling activator as an active ingredient.

In addition, the present disclosure provides use of a three-dimensional neuromuscular organoid for screening for a therapeutic agent for a neuromuscular disease.

In addition, the present disclosure may provide use of a three-dimensional neuromuscular organoid manufactured by the manufacturing method for a neuromuscular organoid for screening for a therapeutic agent for a neuromuscular disease, the manufacturing method including the following processes:
(S0) culturing hPSCs derived from a subject in a medium containing GSK-3 inhibitor and FGF to differentiate into bipotent NMPs;
(S1) diluting the bipotent NMPs, followed by dispensing, to form embryoid bodies; and
(S2) culturing the embryoid body in a medium containing a sonic hedgehog (SHH) signaling activator to produce a three-dimensional neuromuscular organoid.

In addition, the present disclosure provides use of a neuromuscular disease model for screening for a therapeutic agent for a neuromuscular disease, the neuromuscular disease model including the three-dimensional neuromuscular organoid manufactured by the manufacturing method for a neuromuscular organoid, wherein the manufacturing method includes the following processes:
(S0) culturing human pluripotent cells (hPSCs) derived from a subject in a medium containing glycogen synthase kinase 3 (GSK-3) inhibitor and fibroblast growth factor (FGF) to differentiate into bipotent neuromesodermal progenitors (NMPs);
(S1) diluting the bipotent NMPs, followed by dispensing, to form embryoid bodies; and
(S2) culturing the embryoid body in a medium containing an SHH signaling activator to produce a three-dimensional neuromuscular organoid.

In the present disclosure, "therapeutic agent" may be used interchangeably with a pharmaceutical composition, but the embodiment is not limited thereto.

The neuromuscular organoid of the present disclosure may be included in a pharmaceutical composition at a concentration1 pg to 30 g w/v%, but the embodiment is not limited thereto.

The present disclosure may provide a pharmaceutical composition including the neuromuscular organoid as an active ingredient.

In the present disclosure, "administration" refers to introduction of the pharmaceutical composition of the present disclosure to a patient by any suitable method, and the route of administration of the composition of the present disclosure may be via various oral or parenteral routes as long as the composition reaches a target tissue.

In the present invention, "prevention" refers to any act of delaying neuromuscular disease by administration of the composition according to the present disclosure, "treatment" refers to any act whereby symptoms of a neuromuscular disease are ameliorated or beneficially altered by administration of the pharmaceutical composition according to the present disclosure, and "amelioration" refers to any act whereby parameters related to a neuromuscular disease, such as symptom severity, are reduced by administration of the composition according to the present disclosure.

In the present disclosure, the pharmaceutical composition may further include suitable carriers, excipients, and diluents commonly used in the manufacture of pharmaceutical compositions.

In the present disclosure, "carrier" may be also referred to as "vehicle", referring to a compound that facilitates addition of a protein or peptide into a cell or tissue. For example, dimethyl sulfoxide (DMSO) is a commonly used carrier that facilitates introduction of many organic substances into cells or tissues of a living organism.

In the present disclosure, "diluent" is defined as a compound that is diluted in water, which not only stabilizes the biological active form of a target protein or peptide, but also dissolves a protein or peptide. A salt dissolved in a buffer solution is used as a diluent in the art. The commonly used buffer solution is phosphate buffered saline, as it mimics the salty state of human body fluids. Buffer salts may control the pH of a solution at low concentrations, and in this regard, a buffer diluent hardly alter the biological activity of a compound. Compounds including azelaic acid used herein may be administered to human patients as such, or as a pharmaceutical composition mixed with other components or suitable carriers or excipients, as in combination therapy.

Furthermore, the pharmaceutical composition according to the present disclosure may be formulated and used in the form of external preparations such as a powder, a granule, a tablet, a capsule, a suspension, an emulsion, a syrup, aerosol, etc., each prepared by conventional methods, as well as sterile injectable solutions. Examples of a carrier, an excipient, and a diluent that may be included in the composition include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. In the case of formulation, a commonly used diluent or excipient, such as a filler, an extender, a binder, a wetting agent, a dissociating agent, a surfactant, and the like, may be used for preparation. Examples of a solid formulation for oral administration includes a tablet, a pill, a powder, a granule, a capsule, and the like, and such a solid formulation may be prepared by mixing the compound with at least one excipient, such as starch, calcium carbonate, sucrose or lactose, gelatin, and the like. Furthermore, in addition to simple excipients, lubricants such as magnesium stearate talc may be used. Examples of a liquid formulation for oral administration include a suspending agent, an oral liquid, an emulsion, a syrup, and the like. In addition to water and liquid paraffin, which are simple diluents commonly used, various excipients, such as a wetting agent, a sweetening agent, a flavoring, a preservative, and the like may be used. Examples of a formulation for parenteral administration includes a sterile solution, a non-aqueous solvent, a suspending agent, an emulsion, a freeze-dried agent, and a suppository. Examples of a non-aqueous solvent and a suspending agent include propylene glycol, polyethylene glycol, vegetable oil such as olive oil, and injectable ester such as ethyl oleate. Examples of a base agent for the suppository includes witepsol, macrogol, tween 61, cacao butter, laurin butter, glycerol, gelatin, and the like.

The pharmaceutical composition of the present disclosure may be administered orally or parenterally, and preferably parenterally. Examples of the parenteral administration include intramuscular injection, intravenous injection, subcutaneous injection, intraperitoneal injection, local injection, transdermal injection, and the like.

The appropriate dosage of the pharmaceutical composition of the present disclosure may be prescribed variably based on factors such as a formulation method, an administration method, the age, weight, gender, and pathological condition of a patient, diet, an administration time, an administration route, an excretion rate, and responsiveness.

The pharmaceutical composition of the present disclosure may be prepared in a unit dosage form by formulation using a pharmaceutically acceptable carrier and/or excipient, or may be prepared by filling a pharmaceutically acceptable carrier and/or excipient in a large-capacity container, according to methods that can be easily carried out by those skilled in the art to which the present disclosure pertains. Here, the formulation may be in the form of a solution in an oily or aqueous medium, a suspension, an emulsion, an extract, a powder, a granule, a tablet, or a capsule, and a dispersant or a stabilizer may be additionally added thereto.

The terms used in the present disclosure have been selected from general terms that are currently widely used in consideration of their functions in the present disclosure. However, these terms may vary depending on the intent of those skilled in the art, case law, the emergence of new technologies, etc. Furthermore, in certain cases, the terms may be arbitrarily selected by the applicant, and in such instances, the meaning of the terms will be described in detail in the corresponding description section of the description of the disclosure. Therefore, the terms used in the present disclosure should be defined based on their meaning and the overall context of the present disclosure, rather than merely by their names.

The terms "first", "second", etc. may be used herein to describe various elements, but these elements should not be limited by these terms. These terms are only used to distinguish one element from another element. For example, a first element may be named a second element, and similarly, a second element may also be named a first component, without departing from the scope of the present disclosure. The term "and/or" includes a combination of plural-related items listed or any combination of plural-related items listed.

Throughout the specification of the present disclosure, when a portion is said to "include" an element, another element may be further included, rather than excluding the existence of the other element, unless otherwise described. As used throughout the specification of the specification of the present disclosure, the terms "about," "substantially," and the like are used in the sense of at or near the numerical value when manufacturing and material tolerances inherent in the meaning referred to are given, and are intended to prevent unscrupulous infringers from unfairly exploiting the present disclosure where exact or absolute values are stated to aid understanding of the present disclosure.

Throughout the specification of the present disclosure, the term "combination thereof" included in the expressions in the Makush format refers to a mixture or combination of one or more selected from the group consisting of the elements described in the expressions in the Makush format, and refers to include one or more selected from the group consisting of the elements.

The aforementioned contents may be equally applicable to, although not limited to, the medium composition for culturing the neuromuscular organoid, the manufacturing method for the neuromuscular organoid, the three-dimensional neuromuscular organoid, the neuromuscular disease model, the screening method for a therapeutic agent for a neuromuscular disease, use of the medium composition for culturing the neuromuscular organoid, use of the composition, use for screening for a therapeutic agent for a neuromuscular disease, and use for screening for a therapeutic agent for prevention or treatment of a neuromuscular disease, but the embodiment is not limited thereto.

### Mode for the Invention

Hereinafter, preferable examples are presented to help understanding of the present disclosure. However, examples below are only presented only for easier understanding of the present disclosure, and the contents of the present disclosure are not limited by these examples.

### Experimental Examples. Experiment materials and methods

### Experimental Example 1. Human induced pluripotent stem cells (hiPSCs) and maintenance thereof

hiPSCs lines with SOD1 mutations derived from ALS patients and isogenic control substances were obtained from WiCell (WC035i-SOD1-D90A; WC035i-SOD1-D90D), and use of these substances in the present example was approved by the Institutional Review Board (SKKU 2023-10-041) of Sungkyunkwan University. The cells were maintained in mTeSR1 Plus medium (Stem Cell Technologies) of Matrigel Growth Factor Reduced Basement Membrane Matrix(Corning) in a 5 % CO₂ incubator at 37 °C. For each 6-well cell culture plate (Corning), wells were coated at least 2 hours prior to use with 0.5 mg of Matrigel dissolved in DMEM/F12. The cells were passaged every 3 to 4 days using Versene solution (Gibco). Initially, the cells were rinsed with Versene, and new Versene was added to the cells. The incubation time was optimized for each cell line (4 to 5 minutes). On the first day after passage, the medium was supplemented with 10 µM Y-27632(Caymen Chemical). For cryopreservation of hiPSCs, the cells were resuspended in mFreSR (Stem Cell Technologies) and stored in a liquid nitrogen tank. Then, the cell lines were regularly checked for mycoplasma contamination.

### Experimental Example 2. Induction of neural mesodermal progenitor cells from hiPSCs

hiPSCs were cultured under maintenance conditions for at least two passages, until the cells reached about 40 % confluence. The medium used for the maintenance was washed and remove, and then replaced with a pretreatment medium: N2B27 medium supplemented with 3 µM CHIR99021 (Caymen Chemical) and 40 ng/mL of bFGF (Gibco). The next day, a fresh pretreatment medium was replenished and maintained for 24 to 36 hours until the cells were dissociated for embryoid body formation. N2B27 medium is a 1:1 mixture of DMEM/F12 (Gibco) supplemented with 1 x N2 (Gibco) and a neurobasal medium (Gibco) supplemented with 1 x B27 (Gibco), 1 x Glutamax (Gibco), 0.1 mM beta-mercaptoethanol (Simga), and 1 x Pen-Strep antibiotic (Welgene).

### Experimental Example 3. Production of hiPSC-derived neuromuscular organoid

The cell morphology was monitored until the edge of colonies showed signs of possible pluripotency. Once prepared, the cells were gently separated into single cells by culturing the cells in Accutase at 37 °C for 2 minutes. The single cells were counted and resuspended in supplemented with N2B27 medium 50 µM Y-27632, 10ng/mL bFGF, 2ng/mL IGF-1(R&D Systems), and 2ng/mL HGF(R&D Systems). Here, the cell density was 20,000 cells/mL. 100 uL of the medium containing 2,000 cells was seeded into each well of an Ultra-Low Attachment 96-well plate (Corning) using a multichannel pipette. The plate was centrifuged at 350 x g for 2 minutes and placed in a 5 % CO₂ incubator at 37 °C for agglutination. On Day 2, half (50 µL) of the original medium was removed and 100 µL of fresh N2B27 medium supplemented with 2 ng/mL IGF-1 and 2 ng/mL HGF was replenished per well. Pipetting was performed in a steady and slow manner to remove the medium from the well without disturbing organoids. On Day 4, half (75 µL) of the original medium was removed and 100 µL of fresh N2B27 medium without growth factors was replenished per well. To activate the SHH signaling pathway, purmorphamine (1µm) or SAG (1µm) was added to the N2B27 medium. From this point onward, new N2B27 medium was replenished every other day. On Day 10, the organoids were collected from the 96-well plat and transferred to two 60 mm Petri dishes containing N2B27 medium. The dishes were placed in an orbital shaker rotating at 70 rpm inside a 5 % CO₂ incubator at 37 °C. On Day 30, the organoids from each 60 mm Petri dish were transferred to a 100 mm dish containing N2B27 medium. The process of replenishing the medium for the organoids in the Petri dish was performed by tilting the Petri dish to collect the organoids to one side of the dish. After collecting the organoids on the bottom without floating organoids, the used medium was carefully aspirated and replenished with fresh medium until the remaining medium was sufficient to cover the organoids. Unless too many cells were detached from the organoids and visible under a microscope, the organoids were cultured in the same Petri dish for the entire period. To prevent damage to the organoids during transport. the pipette tips were cut off with sterile scissors. The organoids could be maintained indefinitely on the surface without signs of cell death when fresh medium was regularly replenished.

### Experimental Example 4. Brightfield microscope and live imaging

The brightfield images were acquired using a Nikon Eclipse Ti2 inverted microscope. The organoids were imaged directly in the wells of a Ultra-Low Attachment 96-well plate or in a 35 mm Petri dish containing N2B27 medium. For living imaging, the AVI acquisition option in Leica software was utilized (25 frames per second, 1 to 5 minutes duration).

### Experimental Example 5. Contraction analysis

To quantify muscle contraction such as spasm, live video was captured at 20x zoom in the contraction area. The acquired video using ImageJ software was set as a threshold value to define the organoid boundaries and generate a binary stack. The region of interest (10x50 pixels) was defined, and the region displacement was normalized to the initial region and plotted over time.

To quantify synchronized contractions, videos of the entire organoids were acquired, and the contraction frequency was manually counted. To activate motor neurons, 50 µM glutamate (Sigma-Aldrich) was added to the medium. Then, to inhibit acetylcholine receptors, 10 µM Curare(Sigma-Aldrich) was added to the medium.

### Experimental Example 6. Cryosections

The organoids were fixed in 4% PFA overnight at 4 °C. On the next day, the PFA solution was washed off, and the organoids were transferred to a 30% sucrose solution in PBS and stored overnight at 4 °C. After the organoids settled to the bottom of the tube, the organoids were cultured at room temperature for 30 minutes in a 1:1 mixture of 30 % sucrose solution and OCT. Then, the organoids were washed three times in fresh OCT, and placed into the Tissue-Tek Cryomold. The sample was frozen in isopentane cooled with liquid nitrogen and cut into a 10 µm-thick sections using a cryostat. The thinly sliced sections were placed on translucent microscope slides and stored at -80 °C until ready for histochemistry.

### Experimental Example 7. Immunofluorescence

The organoids were fixed in 4% PFA overnight at 4 °C for whole-mounting staining. The sample was permeabilized in 0.5% Triton X-100 for 5 minutes and blocked in PBS containing 3 % bovine serum albumin and 0.1 % Tween-20 at room temperature for 3 hours. Then, the sample was cultured overnight at 4 °C with primary antibodies diluted in a blocking solution. On the next day, the primary antibodies were washed with PBT (0.1 % Tween-20) and cultured at room temperature for 1 hour with secondary antibodies diluted in a blocking solution. The secondary antibodies were washed with PBT, and the resulting sample was placed in PBS containing 80 % glycerol. The confocal images were acquired using the ZEISS LSM710 and CYTATION-C10 (Agilent). The primary antibodies used in the present example are listed in Table 1.

**[Table 1]**

| **Antigen** | **Cat No.** | **Manufactuer** |
|---|---|---|
| BRACHYURY | AF2085 | R&D SYSTEMS |
| ChAT | AB144P | Sigma-Aldrich |
| GFAP | 13-0300 | Invitrogen |
| MY-32 (Fast MHC) | MA5-11748 | Invitrogen |
| MYH3 (eMHC) | F1.652 | DSHB |
| Neurofilament | 801601 & 837904 | BioLegend |
| S100B | Ab41548 | abcam |
| SOX2 | Ab97959 | abcam |
| SV2A | SV2 | DSHB |
| TUJ1 | Ab18207 | abcam |
| TUJ1 | T8660 | Sigma-Aldrich |

For the cryosections, the sample slides were washed with PBS to remove OCT, and post-fixed in 4 % PFA at room temperature for 15 minutes. The same steps described above were performed, from the permeabilization to the immunolabeling. The samples were mounted with Mowiol and imaged using ZEISS LSM710 and CYTATION-C10.

### Experimental Example 8. Real-time quantitative reverse transcription PCR

RNA was isolated using the easy-BLUE Total RNA extraction kit (iNtRON) according to the protocol of the manufacturer. Then, cDNA synthesis was performed using 0.5 mg of RNA using PrimeScript RT reagent (TaKaRa) according to instructions of the manufacturer. For real-time quantitative reverse transcription PCR (qRT-PCR), a PCR mixture was prepared using the TB Green Premix Ex Taq II (TaKaRa). and the reaction was performed using the Thermal Cycler Dice Real Time System III (TaKaRa). The gene expression values were normalized to GAPDH levels. The sequences of primers used in the present example are listed in Table 2.

**[Table 2]**

| **Gene** | | **5' to 3'** |
|---|---|---|
| **CDON** | Forward | GGCATGATGGGAATCTCCGT **(SEQ ID NO: 1)** |
| | Reverse | AGGTGCCAAGTCTGAACTCAC **(SEQ ID NO: 2)** |
| **CHAT** | Forward | GTGGCTCAGAACAGCAGCATCA **(SEQ ID NO: 3)** |
| | Reverse | CCTCACTGAGACGGCGGAAATT **(SEQ ID NO: 4)** |
| **CHRNA1** | Forward | CCGAGGTGAAAAGTGCCATCGA **(SEQ ID NO: 5)** |
| | Reverse | TCCGAGGAGTATGTGGTCCATC **(SEQ ID NO: 6)** |
| **GAPDH** | Forward | GTCTCCTCTGACTTCAACAGCG **(SEQ ID NO: 7)** |
| | Reverse | ACCACCCTGTTGCTGTAGCCAA **(SEQ ID NO: 8)** |
| **MYH2** | Forward | CACCTGGATGATGCTCTCCG **(SEQ ID NO: 9)** |
| | Reverse | TGATCAGGCTGGTGTTCTGG **(SEQ ID NO: 10)** |
| **MYH4** | Forward | AGCAATAGTGCAGAGCAGGG **(SEQ ID NO: 11)** |
| | Reverse | AGAGGCCCGAGTAGGTGTAG **(SEQ ID NO: 12)** |
| **MYH7** | Forward | CTTGAGTAGCCCAGGCACAG **(SEQ ID NO: 13)** |
| | Reverse | CGAGACACGATCTTGGCCTT **(SEQ ID NO: 14)** |
| **OLIG2** | Forward | ATGCACGACCTCAACATCGCCA **(SEQ ID NO: 15)** |
| | Reverse | ACCAGTCGCTTCATCTCCTCCA **(SEQ ID NO: 16)** |
| **RPL32** | Forward | ACAAAGCACATGCTGCCCAGTG **(SEQ ID NO: 17)** |
| | Reverse | TTCCACGATGGCTTTGCGGTTC **(SEQ ID NO: 18)** |
| **SHH** | Forward | CCAAAAAGCTGACCCCTTTA **(SEQ ID NO: 19)** |
| | Reverse | GCTCCGGTGTTTTCTTCATC **(SEQ ID NO: 20)** |
| **TUBB3** | Forward | AGTGATGAGCATGGCATCGA **(SEQ ID NO: 21)** |
| | Reverse | AGGCAGTCGCAGTTTTCACA **(SEQ ID NO: 22)** |

### Experimental Example 9. Quantification and statistical analysis

To measure the organoid size, ImageJ software was used to automatically generate binary images from the brightfield images, and the total area of each organoid was quantified through particle analysis. To quantify the Fast MHC expression area, the stained area was calculated using the Image Statistics utility of the Gen5 software (Agilent Technologies) and divided as the DNA staining area. To quantify the number of AChR clusters, an object mask was generated using the cell analyzer of the Gen5 software, and the number of objects was counted across the entire field of view. The size of individual AChR cluster was recorded per individual area in the calculated metrics of the cell analysis device.

All data were reported as mean ± standard error of the mean. The statistical significance was calculated using Student's t-test, and differences were considered statistically significant when the P value was less than 0.05. After analyzing the data using one-way ANOVA for comparison between several groups, Tukey's multiple comparison test was performed using Prism 9.1.0, GraphPad.

### Examples. Experiment results

### Example 1. Manufacture of neuromuscular organoid

### Example 1-1. Pretreatment for inducing differentiation of hiPSCs into NMPs

hiPSCs were cultured in a Matrigel-coated tissue culture-treated 6-well plate. In addition, to induce differentiation of stem cells into bipotent NMPs, pretreatment was performed at about 40 % confluency. Here, 3 µm CHIR99021 and 40 ng/mL bFGF were added to N2B27 medium for the pretreatment.

### Example 1-2. Embryoid body formation where cells were seeded at optimal density

To form embryoid bodies after 24 hours of the pretreatment, the cells were treated with Accutase for 2 minutes at 37 °C to dissociate the cell-to-cell binding. The number of separated cells was counted using a hemocytometer, and the cell concentration was diluted in aggregation medium to contain 2000 cells per 100 µL. Here, the aggregation medium was prepared by adding 50 µM Y-27632, 10 ng/mL bFGF, 2 ng/mL IGF-1, and 2 ng/mL HGF to N2B27 medium. The cells diluted in the aggregation medium were dispensed into each well of an Ultra-Low Attachment round bottom 96-well plate at 100 µL per well, resulting in about 2000 cells per well. After the cells were centrifuged using a centrifuge and collected at the bottom of the wells, the collected cells were cultured in a 37 °C, 5 % CO₂ incubator.

### Example 1-3. Formation of neuromuscular organoids cultured in medium supplemented with purmorphamine

On the second day (Day 2) of the embryoid body formation, 50 µL of medium was removed from each well, and 100 µL of N2B27 medium supplemented with 2 ng/mL IGF-1 and 2 ng/mL HGF was dispensed into each well.

On the fourth day (Day 4) of the embryoid body formation, 80 µL of medium was removed from each well, and 100 µL of N2B27 medium supplemented with 1 µM purmorphamine was dispensed into each well. Subsequently, the cells were cultured in N2B27 medium supplemented with 1 µM purmorphamine until Day 20. The medium was replaced every two days. As the embryoid bodies elongated from this point onward, the precursor cells of the nervous tissue and muscular tissue were spatially separated.

On the tenth day (Day 10) of the embryoid body formation, the organoids from each 96-well plate were transferred and divided into two 60 mm Petri dishes. The transferred organoids were cultured in an orbital shaker at 75 rpm in a 5 % CO₂ incubator at 37 °C.

The characteristics of the neuromuscular organoids of the present disclosure, manufactured according to Example 1 (FIG. 1), were confirmed as shown in FIG. 2. The specific characteristics were confirmed as follows.

### Example 2. Confirmation of morphological characteristics of neuromuscular organoids

### Example 2-1. Embryoid body formation by pretreatment methods and confirmation of morphological characteristics of neuromuscular organoids

Specifically, the formation of embryoid bodies according to pretreatment methods and the induction of neuromuscular morphogenesis were compared (FIGS. 3A and 3B). FIG. 3A shows representative images of successfully formed embryoid bodies (middle) and failed embryoid bodies (left), along with the successful embryoid body formation rate (right) according to the pretreatment method, and FIG. 3B shows representative images of successful neuromuscular morphogenesis based on elongation (middle) and failed morphogenesis (left), along with the successful morphogenesis rate according to the pretreatment method (right).

Conventionally, the pretreatment was performed simultaneously with cell passage, but in the present disclosure, the pretreatment was performed through medium replacement during the cell culture process. Furthermore, conventionally, the pretreatment was performed after isolating stem cells into single cell state via Accutase treatment. However, in the present disclosure, the pretreatment was performed while stem cells were in a colony state, confirming that this approach was more suitable for inducing successful formation and differentiation of embryoid bodies (FIGS. 3A and 3B).

That is, the pretreatment method performed in the present disclosure in the colony state without separating cells into the single cell state was found to increase the rate of successful embryo formation and excellent neuromuscular morphogenesis.

### Example 2-2. Confirmation of excellent morphological characteristics of neuromuscular organoids over time

The neuromuscular organoid was confirmed to be successfully formed based on the morphogenesis of the neuromuscular organoid formed by the protocol of the present (FIG. 4). Specifically, the successful neuromuscular organoid formed both nervous tissue and skeletal muscle tissue within a single organoid. The two tissues were separated, forming a "peanut"-like shape, and each tissue tended to develop on the opposite side (FIG. 4).

The two tissues could be distinguished by the relatively dark color of the skeletal muscle tissue. Such a distinctive color of the skeletal muscle became more clear as the organism matured. On the nervous tissue side, the neural epidermis appeared, along the entire surface of the nervous tissue, as several small rose-like structures or as the continuous neural epidermis. For reference, there were instances where the two tissues were not completely separated from one another. In these cases, the skeletal muscle portion was found as a small protrusion, and the protrusion was confirmed to be surrounded by the nervous tissue.

### Example 3. Confirmation of neuromuscular organoid biomarkers

### Example 3-1. Confirmation of expression of NMP marker according to pretreatment

The differentiation of hiPSCs into bipotent NMPs was achieved by the pretreatment, as confirmed by the expression of NMP markers, SOX2 and BRA (FIG. 5).

As a result, the induction into NMPs was referred by the co-expression of SOX2 and BRA. In addition, prior to the pretreatment, the hiPSCs expressed only SOX2, which is one of the pluripotent factors, along with OCT4 and NANOG (FIG. 5, left). The cells expressing SOX2 and BRA simultaneously appeared about 24 hours after the pretreatment (FIG. 5, middle). Within one day after aggregation, the embryoid bodies produced by the pretreated hiPSCs also co-expressed SOX2 and BRA, suggesting that most cells successfully differentiated into NMPs (FIG. 5, right).

### Example 3-2. Confirmation of markers associated with neural ectoderm formation in neuromuscular organoid

The SHH signaling pathway is a key factor for ventralization of the spinal cord neurons, and in this regard, purmorphamine as the SHH signaling agonist was added to the organoids to induce development of the neuromuscular organoids into motor neurons. During the embryogenesis process, SHH was expressed in the notochord when neural stem cells emerged to form the neural plates. On Day 4, the neuromuscular organoids began expressing the neural stem cell marker (e.g., SOX2) in one of the organoid tails. Furthermore, starting on Day 7, the expression of a neural marker (e.g., TUJ1) was confirmed in the region where the neural stem cell marker (e.g., SOX2) was expressed (FIG. 6).

On the 4th day of organoid formation, the formation of neuroectoderm was confirmed by local expression of SOX2 in the area where nervous tissue is formed (left panel of FIG. 6). From the 7th day of organoid formation, the differentiation of neural stem cells into neurons was confirmed by expression of TUJ1 (middle panel of FIG. 6). Based on this, it was decided to proceed treatment with the SHH signaling agonist from the 4th day of organoid formation, and as neural stem cells appeared on Day 4 of neuromuscular organoid formation, it was decided to proceed treatment with purmorphamine for various durations from Day 4 (from Day 4 to Day 6, Day 4 to Day 10, Day 4 to Day 20).

### Example 3-3. Confirmation of markers associated with neuromuscular junction formation in neuromuscular organoid

The successful formation of neuromuscular organoids according to the protocol of the present disclosure was confirmed through staining with TUJ1 as a neuron marker and Fast MHC as a mature skeletal muscle marker (FIG. 7B). The panel on the right (yellow border) shows a magnified view of the skeletal muscle region of the neuromuscular organoid, showing axons extending from the nervous tissue into the skeletal muscle tissue and forming neuromuscular junctions.

In addition, the successful formation of nervous tissue and skeletal muscle tissue was confirmed through immunolabelling. The neuromuscular organoids on Day 20 formed extensive neurites visualized by TUJ1 (FIG. 7A). eMHC which labels developing muscle fibers was detected in the same organoid surrounded by TUJ1 labeling. Upon detailed examination of skeletal muscle tissue, myotubes were found with neurites extending from peripheral nervous tissue. Fast myosin heavy chain (MYH2) representing formation of mature muscle fibers appeared in the skeletal muscle portion of the neuromuscular organoid on Day 40 (FIG. 7B). The higher magnification of the skeletal portion revealed more extensive neurites intertwined with muscle fibers. Sarcomere of individual muscle fiber was observed in the neuromuscular organoid on Day 60 (FIG. 7C).

### Example 4. Confirmation of SHH signaling activation in neuromuscular organoid by purmorphamine

On the 20th day, the neuromuscular organoids were collected and quantitatively evaluated by qRT-PCR for the effect of purmorphamine.

As a result, it was confirmed that SHH expression was significantly induced by purmorphamine in proportion to the treatment period (FIG. 8). This demonstrated that treatment with purmorphamine activated the SHH signaling in the organoids.

Specifically, it was confirmed that treatment with purmorphamine significantly increased the SHH expression, whereas expression of CDO, which is a co-receptor factor for SHH, decreased inversely proportional to the SHH expression due to a negative feedback.

### Example 5. Confirmation of differentiation into motor neurons in neuromuscular organoids by purmorphamine

Whether differentiation into motor neurons was induced by treatment with purmorphamine was confirmed by expression levels of motor neuron markers (FIG. 9). The expression of both OLIG2 as a motor neuron progenitor marker and ChaT as a motor neuron marker was significantly increased in the organoid group treated with purmorphamine for the longest period (Day 4 to Day 20).

This showed that maintaining the SHH signaling from Day 10 to Day 20 of the organoid formation is important for the formation of motor neurons in the neuromuscular organoids and that the activation of SHH signaling by purmorphamine promotes motor neuron development in the neuromuscular organoids.

In addition, an increase in the expression of the motor neuron marker was accompanied by a decrease in the expression of general neuron markers (e.g., TUBB3) in the organoids treated with purmorphamine. This suggests that the longer the treatment with purmorphamine, the greater the overall decrease in the expression of nervous tissue markers, and thus the effect of purmorphamine on differentiation into motor neurons may actually be greater than the results shown in FIG. 9.

That is, it was confirmed that the expression of motor neuron markers increased in the neuromuscular organoids of the present disclosure (FIG. 9), and that the treatment with purmorphamine reduced nervous tissue in the organoids while promoting proliferation of skeletal muscle tissue.

### Example 6. Confirmation of effect of purmorphamine on increasing formation of neuromuscular junction in neuromuscular organoid

The frequency of neuromuscular junction formation following treatment with purmorphamine was confirmed using α-bungarotoxin (α-BTX), resulting in an increased trend (FIG. 10).

This suggests that the neuromuscular junction was formed in the neuromuscular organoid of the present disclosure.

### Example 7. Confirmation of morphological characteristics of neuromuscular organoid upon purmorphamine

### Example 7-1. Confirmation of increase in muscle tissue portion according to period of treatment with purmorphamine

After adding purmorphamine to the culture medium starting on Day 4 of organoid formulation, differences in organoid morphology based on treatment duration were compared. As a result, it was confirmed that the organoid size generally increased with longer treatment period with purmorphamine, particularly in the muscle tissue portion (FIG. 11). In addition, after adding purmorphamine to the culture medium starting on Day 4 of organoid formulation, differences in organoid morphology based on treatment duration were compared on Day 50. As a result, it was confirmed that the longer the treatment period with purmorphamine, the smaller the nervous tissue became and the larger the skeletal muscle tissue grew.

The results shown in FIG. 11 suggest that the increase in size induced by purmorphamine was primarily due to the growth of skeletal muscle tissue rather than nervous tissue, when considering that nervous tissue portions and skeletal muscle portions of the neuromuscular organoids were distinguishable by color under a brightfield microscope.

### Example 7-2. Confirmation of increase in overall size by purmorphamine

One of the first noticeable effects of purmorphamine on the neuromuscular organism was an increase in size. Organoids increased in size proportionally to the duration of purmorphamine treatment (FIG. 12A).

Such effects on the organoid size was also observed when the neuromuscular organoid was treated with Smoothened agonist (SAG), another type of the SHH signaling agonist. Specifically, when comparing the size of organoids following SAG treatment, it was confirmed that the organoid size increased upon the SAG treatment compared to a control group (FIG. 12B).

That is, the present disclosure suggests that the overall size of organoids was increased, thereby promoting proliferation of skeletal muscle cells in the neuromuscular organoids (FIGS. 11 and 12).

### Example 8. Confirmation of effect of purmorphamine in promoting muscle tissue maturation in neuromuscular organoid

### Example 8-1. Confirmation of increased expression of mature muscle fiber markers

The increased expression of mature muscle fiber markers by the purmorphamine treatment was confirmed by qRT-PCT (FIG. 13). Such results were confirmed by qRT-PCR analysis on the neuromuscular organoids on Day 20 treated with purmorphamine for various durations, as the expression of MHC was significantly increased by purmorphamine (FIG. 13).

Specifically, it was confirmed that the expression of myosin heavy chain 2 (MYH2) and myosin heavy chain 7 (MYH7), which are a marker for fast twitch oxidative (IIA type) muscle fiber and a marker for slow twitch (I type) muscle fiber, respectively, was induced by treatment with purmorphamine, and that the expression of MYH4, which is a muscle fiber indicator for fast twitch glycolytic (IIB type) muscle fiber.

### Example 8-2. Confirmation of early expression of mature muscle fiber markers

The onset of the expression of fast MHC, which is expressed specifically in mature muscle fiber, was confirmed to be advanced compared to the control group (FIG. 14). That is, the present disclosure suggests that the method described herein promotes the maturation of skeletal muscle cells in neuromuscular organoids.

### Example 9. Confirmation of excellent effect of purmorphamine on muscle tissue contraction in neuromuscular organoid

### Example 9-1: Confirmation of early contraction of muscle tissue

As the maturation of muscle tissue was promoted, it was confirmed on Day 14 of the organoid formation that the time at which muscle tissue contraction was observed was advanced compared to the control group (FIG. 15).

In addition, it was confirmed that the timing of glutamate-induced synchronous contraction observed in organoids treated with purmorphamine was significantly accelerated. This provides further evidence of the maturation of skeletal muscle tissue induced by purmorphamine.

Specifically, organoids in the control group and organoids treated with purmorphamine for a short period (4 to 6 days) did not respond to glutamate, but organoids treated with purmorphamine for a long period (4 to 10 days; 4 to14 days) exhibited synchronous contraction in response to glutamate treatment on Day 14 (FIG. 15). Overall, such data suggest that purmorphamine promoted the growth and maturation of skeletal muscle in neuromuscular organoids.

### Example 9-2. Confirmation of synchronized contraction of entire skeletal muscle tissue

In the neuromuscular organoids of the present disclosure, synchronized contraction of the entire skeletal muscle tissue, which is more similar to contraction of actual human muscle than spontaneous contraction observed in existing neuromuscular organoids, was observed (FIG. 16). That is, it was confirmed that synchronized contractions resembling the actual contractions of skeletal muscle tissue in the human body occurred, which had not been observed in conventional neuromuscular organoids, due to the promoted proliferation of skeletal muscle cells.

Specifically, previous studies described spontaneous contraction of skeletal muscle tissue formed in neuromuscular organoids at Day 40 to 50 as further evidence of successful formation of skeletal muscle. Such small and rapid spasm-like contraction was also observed in the neuromuscular organoids formed in the present disclosure. To quantify such contraction, the skeletal muscle portion of the neuromuscular organoid was imaged in real time at high magnification. Then, the region displacement within the region of interest in the acquired image was plotted over time. The contractile movement of organoids tracked over time showed viability among organoids (FIG. 17).

However, unlike spasms of skeletal muscle described in previous studies, much stronger synchronous contraction of the entire skeletal muscle in the neuromuscular organoid was also observed.

Specifically, the muscle tissue of the neuromuscular organoid was enlarged and videotaped, and the area change of the muscle tissue within the region of interest (ROI) over time was tracked to chart the contraction of the muscle tissue. The part pointed by the arrow in 'Organoid 3' indicates overall contraction of the entire muscle tissue.

That is, it was confirmed that the proliferation and maturation of skeletal muscle cells in neuromuscular organoids caused synchronized contraction resembling the actual contraction of skeletal muscle tissue in the actual human body occurred, which had not been observed in conventional neuromuscular organoids.

### Example 10. Optimization of neuromuscular organoid protocol

### Example 10-1. Confirmation of optimal cell seeding density after pretreatment

The problem with the prior art is that it is difficult to find the appropriate seeding density of cells after pretreatment. When the cells were seeded at the recommended seeding density (75,000 to 125,000 /cm²) as in the prior art, the cells often became overly confluent after 3 days in the pretreatment medium. On the other hand, reducing the initial seeding density results in the production cells with inferior quality, due to the fact that hiPSCs prefer to form colonies to ensure survival (Rivera, T., et al., Human-Induced Pluripotent Stem Cell Culture Methods Under cGMP Conditions. Current Protocols in Stem Cell Biology, 2020. 54(1): p. e117.). These difficulties resulted in inconsistent outcomes for healthy embryoid body formation and successful organoid formation.

Therefore, to overcome these problems, the present inventors tested other procedures for the pretreatment of hiPSCs. Other papers (Olmsted, Z.T. and J.L. Paluh, Co-development of central and peripheral neurons with trunk mesendoderm in human elongating multi-lineage organized gastruloids. Nature Communications, 2021. Hereinafter referred to as Olmsted and Paluh) described a protocol for generating elongating multi-lineage organized (EMLO) gastruloids using the same pretreated medium containing CHIR99021 and bFGF to form tissues derived from all three germ layers. The hiPSCs were pretreated by simply replacing the culture medium with the pretreated medium when the cell confluency reached about 60 %. This procedure for the hPSC pretreatment was combined with the rest of the protocol for neuromuscular organoids described by Martins et al. Controlling the cell confluency has become significantly easier, resulting in more consistent results.

### Example 10-2. Determining optimal cell number for production of single-cell embryoid body

The number of starting cells for producing single-cell embryoid bodies that determine the size was optimized. The embryoid bodies of the prior art (Martins et al.) were produced with 4,500 to 9,000 cells depending on the cell line used. When this number of cells was used for single-cell embryoid bodies, the size of the embryoid bodies on Day 1 was significantly greater than the size of embryoid bodies described by Martins et al. (9,000 cells: diameter of up to 500 µm; 4,500 cells: diameter of up to 250 µm) (FIG. 18). Such large embryoid bodies failed to successfully form neuromuscular organoids and remained rounded, suggesting that the size/number of cells per embryoid body is another parameter requiring optimization for successful organoid formation.

By comparing various numbers of cells for producing single-cell embryoid bodies, 2000 cells (diameter of approximately 150 µm) were calculated as the optimal number for the formation of neuromuscular organoids (FIG. 3A, middle panel).

### Example 11. Identification of morphological characteristics of skeletal muscle parts associated with reproduction of neuromuscular organoid formation

Acetylcholine receptor clusters were visualized by α-bungarotoxin (αBTX) labeling (FIG. 19A). The widespread presence of satellite cells within skeletal muscle regions was identified by PAX7 expression (FIG. 19B). Meanwhile, only a small subset of motor neurons was identified near the boundary between the nervous tissue and the skeletal muscle tissue by the expression of choline acetyltransferase (ChAT) (FIG. 19C). Such characteristics suggest that the formation of neuromuscular organoids was successfully reproduced through protocol optimization.

### Example 12. Identification of cause of synchronous contraction formed in neuromuscular organoids

This type of contraction (hereinafter referred to as 'synchronous contraction' to distinguish it from contraction such as spasm) was infrequent, occurring about once every two minutes (FIGS. 20A and 20B). To confirm whether the synchronous contraction observed in the neuromuscular organoids is regulated by motor neurons that activate skeletal muscle fibers through the neuromuscular junction, changes in contraction velocity were monitored following treatment with glutamate and curare. Following treatment with an excitatory neurotransmitter, glutamate, the average synchronous contraction velocity increased slightly without statistical significance. More importantly, when the organoids were treated with curare, which is a toxin that inhibits the function of acetylcholine receptors in muscle fibers, the synchronous contraction was completely stopped.

This suggests that the synchronous contraction results from muscle fibers activated by acetylcholine receptors at the neuromuscular junction.

### Example 13. Confirmation of markers associated with synchronous contraction in neuromuscular organoid

Neuromuscular organoids exhibiting synchronous contraction were collected separately from the remaining neuromuscular organoids to investigate the differences between the synchronically contracting neuromuscular and the remaining non-contracting neuromuscular organoids. According to qRT-PCR analysis, the organoids exhibiting synchronous contraction were found to express lightly high levels of MHC in all isoforms tested (FIGS. 21A to 21C). Meanwhile, there was no significant difference in the expression levels of neural markers between the two groups (FIGS. 21D and 21E). In addition, compared to the remaining organoids, the expression of neuromuscular junction-related genes was significantly increased in the organoids exhibiting synchronous contraction (FIGS. 21F and 21G). Such data suggest that synchronous contraction in the neuromuscular organoids results from the development and maturation of skeletal muscle within the organoids.

### Example 14. Analysis of morphological characteristics of neuromuscular organoids suitable for personalized medicine in amyotrophic lateral sclerosis (ALS)

The most interesting application of neuromuscular organoids lies in their use in personalized medicine. Neuromuscular organoids generated from patient-derived hiPSCs are able to model disease causes and select appropriate treatment options in a patient-specific manner (FIG. 22). To this end, amyotrophic lateral sclerosis(ALS) patient-derived SOD1 mutant hiPSC line and an isogenic control hiPSC line with corrected SOD1 gene were obtained and used to generate neuromuscular organoids for proof-of-concept studies. Organoids generated from the SOD1 mutant hiPSC line showed abnormal morphology in relatively small skeletal muscle portions and exhibited unidentifiable morphological characteristics not observed in organoids derived from the control hiPSC line (FIGS. 23A and FIG. 23B).

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

### Industrial Applicability

The neuromuscular organoid of the present disclosure is cultured in a medium containing a composition including a sonic hedgehog signaling agonist as an active ingredient, thereby not only promoting a process of differentiating neurons constituting the neuromuscular organoid into motor neurons, but also promoting the development and maturation of muscle fibers. The interactions between motor neurons and muscle tissues through a neuromuscular junction are essential in terms of the cause and treatment of degenerative neuromuscular diseases, and thus, the neuromuscular organoid of the present disclosure can be used as a model of neuromuscular diseases (specifically, a degenerative neuromuscular disease model). Therefore, the present disclosure is expected to be also applicable to mechanism studies and drug screening for neurological and muscular diseases, demonstrating industrial applicability.

## Claims

1. A medium composition for culturing a neuromuscular organoid, for three-dimensional culture of pluripotent stem cells (PSCs) into a neuromuscular organoid, the medium composition comprising a sonic hedgehog (SHH) signaling activator as an active ingredient.

2. The medium composition of claim 1, wherein
the SHH signaling activator comprises at least one selected from the group consisting of an SHH signaling agonist and an SHH protein.

3. The medium composition of claim 2, wherein
the SHH signaling agonist comprises at least one selected from the group consisting of purmorphamine and a smoothened agonist (SAG).

4. The medium composition of claim 1, further comprising
at least one selected from the group consisting of a glycogen synthase kinase 3 (GSK-3) inhibitor, a fibroblast growth factor (FGF), a Y-27632, insulin-like growth factor (IGF), and a hepatocyte growth factor (HGF).

5. The medium composition of claim 1, wherein
the neuromuscular organoid comprises at least one selected from the group consisting of a nervous tissue, a skeletal muscle tissue, and a neuromuscular junction.

6. The medium composition of claim 1, wherein
the PSCs are human induced pluripotent stem cells (hiPSCs).

7. A manufacturing method for a neuromuscular organoid, the method comprising the following processes:
(S0) culturing subject-derived human pluripotent cells (hPSCs) in a medium containing glycogen a synthase kinase 3 (GSK-3) inhibitor and a fibroblast growth factor (FGF) to differentiate into bipotent neuromesodermal progenitors (NMPs);
(S1) diluting the bipotent NMPs, followed by dispensing, to form embryoid bodies; and
(S2) culturing the embryoid bodies in a medium containing a sonic hedgehog (SHH) signaling activator to manufacture a three-dimensional
neuromuscular organoid.

8. The manufacturing method of claim 7, wherein,
in process (S0), the hPSCs are in a colony state.

9. The manufacturing method of claim 7, wherein,
in process (S0), the bipotent NMPs express SRY sex-determining region Y-box 2 (SOX2) or BRACHYURY (BRA).

10. The manufacturing method of claim 7, wherein,
in process (S1), a medium composition for diluting the bipotent NMPs contains at least one selected from the group consisting of Y-27632, FGF, insulin-like growth factor (IGF), and hepatocyte growth factor (HGF).

11. The manufacturing method of claim 7, wherein process (S1) is **characterized by** at least one selected from the following group consisting of:
(a) performing process (S1) before the bipotent NMPs form the embryoid bodies; and
(b) dispensing 1500 to 9000 bipotent NMPs.

12. The manufacturing method of claim 7, wherein,
in process (S2), the culturing is orbital shaking culturing.

13. A three-dimensional neuromuscular organoid manufactured by the manufacturing method of any one of claims 7 to 12.

14. The neuromuscular organoid of claim 13, wherein
the organoid expresses at least one selected from the group consisting of neuron-specific class **III** beta-tubulin (TUJ1), fast myosin heavy chain (MHC), SOX2, SHH, α-bungarotoxin (α-BTX), myosin heavy chain 2 (MYH2), and myosin heavy chain 7 (MYH7).

15. The neuromuscular organoid of claim 13, wherein the neuromuscular organoid is **characterized by** at least one selected from the following group consisting of:
(a) decreased expression of Cdo and Tubb3; and
(b) increased expression of Olig2 and choline acetyltransferase (ChAT).

16. The neuromuscular organoid of claim 13, wherein the neuromuscular organoid is **characterized by** at least one selected from the following group consisting of:
(a) promoting differentiation into motor neurons;
(b) increasing the size of muscle fibers; and
(c) maturating muscle fibers.

17. The neuromuscular organoid of claim 13, wherein
the neuromuscular organoid is **characterized by** synchronous contraction.

18. A neuromuscular disease model comprising the neuromuscular organoid of claim 13.

19. The neuromuscular disease model of claim 18, wherein
the neuromuscular disease is a degenerative neuromuscular disease.

20. The neuromuscular disease model of claim 19, wherein
the degenerative neuromuscular disease is selected from the group consisting of spinal muscular atrophy (SMA), amyotrophic lateral sclerosis (ALS), Duchenne muscular dystrophy, and myotonic dystrophy.

21. A screening method for a therapeutic agent for a neuromuscular disease, the method comprising the following processes:
(1) treating a neuromuscular organoid derived from a neuromuscular disease subject manufactured by the manufacturing method of claim 7 with a candidate substance for treating a neuromuscular disease; and
(2) comparing morphological characteristics of the neuromuscular organoid treated with the candidate substance.

22. The screening method of claim 21, further comprising
(3) determining the candidate substance as a therapeutic agent for a neuromuscular disease, when the morphological characteristics of the neuromuscular organoid treated with the candidate substance exhibit morphological characteristics of normal neuromuscular tissue.

23. Use of a medium composition for three-dimensional culture of pluripotent stem cells (PSCs) into a neuromuscular organoid, the medium composition comprising a sonic hedgehog (SHH) signaling activator as an active ingredient.

24. Use of a composition for manufacturing a neuromuscular organoid from pluripotent stem cells (PSCs), the composition comprising a sonic hedgehog (SHH) signaling activator as an active ingredient.

25. Use of the three-dimensional neuromuscular organoid of claim 13 for screening for a therapeutic agent for a neuromuscular disease.
